# EUROPEAN PATENT APPLICATION

(11) **EP 2 899 213 A1**
(43) Date of publication of application: **29.07.2015**
(21) Application number: 14152669.9
(22) Date of filing: 27.01.2014
(51) Int. Cl.: C08B 1/00, C08B 1/06, C08B 11/00, C08B 15/06, C08B 31/00, C08B 31/08, C08B 37/00, C09D 101/08, C09D 101/26, C09D 103/04, C09D 103/08, C09D 105/00, C09J 101/08, C09J 101/26, C09J 103/04, C09J 103/08, C09J 105/00

(54) **Modified polysaccharides, method for their production and their use**

(71) Applicant: BASF SE, 67056 Ludwigshafen (DE)
(72) Inventor: Granström, Mari, 00120 Helsinki (FI); Zajaczkowski-Fischer, Marta, 67141 Neuhofen (DE); Kindler, Alois, 67269 Grünstadt (DE)
(74) Representative: Reitstötter Kinzebach

(57) **Abstract**

The present invention relates to a method for producing an ether modified polysaccharide, ether modified polysaccharide obtainable by said method and the use thereof. The present invention relates to a method for producing an ethylenically unsaturated polysaccharide, ethylenically unsaturated polysaccharides obtainable by said method, polymers comprising those unsaturated polysaccharides in polymerized form and the use thereof.

## Description

### FIELD OF THE INVENTION

The present invention relates to a method for producing modified polysaccharides carrying substituents bound via ether or amino groups, modified polysaccharide obtainable by said method and the use thereof.

Polyhydroxy-funtionalized polymers are used in many technical fields, e.g. cosmetic and pharmaceutical compositions or coatings for the textile, paper, printing and leather industry. In particular polysaccharides play an important role in cosmetic formulations due to their unique application characteristics. Thus, polysaccharides can act as thickeners, suspending agents, hair conditioners, moisturizers, emulsifiers and emollients. In pharmaceutics, polysaccharides are employed e.g. in wound-healing agents. Nevertheless in many cases the application properties of known polysaccharides still needs improvement. For example, many natural and modified polysaccharides cannot be incorporated into liquid formulations with justifiable effort. There is also a demand to enhance the product performance of synthetic polymers by incorporation of polysaccharides into those polymers for the purpose of conferring the advantageous properties of the polysaccharides at least partly to those polymers. A prerequisite for this is that polysaccharide macromers can be provided which are copolymerizable with the typical monomers of customary polymers used in cosmetics, pharmaceutics etc.

WO 2004/099273 relates to water-soluble or water-dispersible polymers made from monoethylenically-unsaturated saccharide monomers, comprising a polymer of
(A) 5 to 100 wt.% of at least one ester, selected from the group of monoacrylate and monomethacrylate esters of saccharides and saccharide derivatives, wherein the monoester has a content of saccharides or saccharide derivatives multiply esterified with acrylic or methacrylic acid of ≤ 2.5 wt.% and
(B) 0 to 95 wt.% of at least one further monoethylenically-unsaturated monomer,
and which have a mean molecular mass Mw of 5,000 to 2,500,000.

US 2011/0175023 A1 describes a process for chlorinating polysaccharides, which comprises
a1) dissolving a polysaccharide in a solvent system which comprises at least one ionic liquid, and
a2) reacting the polysaccharide with a chlorinating agent.

WO 2011/157777 relates to a water-soluble or water-dispersible copolymer containing units of
a) at least one ethylenically unsaturated monomer comprising a saccharide side group and
b) at least one hydrophilic monomer that is different from (meth)acrylamide,
wherein the percentage by weight of the ethylenically unsaturated monomers comprising a saccharide side group is 5 to 95 wt.%. The copolymers exhibit high affinity to inorganic surfaces or hydrophilic fibers such as cotton and are used in textile detergents as polymers that are able to dissolve dirt and/or prevent graying or minimize the risk of the microbial colonization of materials coated therewith.

It has now been found surprisingly, that modified polysaccharides with advantageous properties can be obtained if a chlorinated polysaccharide is subjected to a functionalization with a compound that is suitable for replacing the chlorine atoms by functional groups with hydrophilic or hydrophobic properties. The resulting modified polysaccharides have an overall good application profile for diverse areas of use, e.g. in personal care and pharmaceutical compositions, compositions used for industrial or institutional or hospital disinfection, compositions used for material protection, etc.

### SUMMARY OF THE INVENTION

According to a first aspect of the present invention there is provided a method for producing a modified polysaccharide, which comprises
a) providing a chlorinated polysaccharide, wherein at least a part or all of the hydroxyl groups have been replaced by chlorine atoms,
b) reacting the chlorinated polysaccharide with at least one compound, selected from compounds of the formula (I)

HX¹-R¹ (I)

wherein
- X¹: is O or NR^{a}, where R^{a} is hydrogen, C₁-C₆-alkyl, C₃-C₈-cycloalkyl or C₆-C₂₀-aryl,
- R¹: is selected from C₁-C₃₀-alkyl, C₃-C₃₀-alkenyl, C₂-C₃₀-hydroxyalkyl, amino(C₁-C₃₀-alkyl), mono-(C₁-C₆-alkyl)amino(C₁-C₃₀-alkyl), di-(C₁-C₆-alkyl)amino(C₁-C₃₀-alkyl), C₃-C₈-cycloalkyl and C₆-C₂₀-aryl,
wherein the alkyl group of C₁-C₃₀-alkyl and C₂-C₃₀-hydroxyalkyl may be interrupted by one or more non-adjacent groups selected from -O-, -S-, -C(=O)- and -N(R^{b})-, where R^{b} is hydrogen, C₁-C₆-alkyl, C₃-C₈-cycloalkyl or C₆-C₂₀-aryl,
wherein the cycloalkyl and aryl groups in each case can be unsubstituted or substituted by 1, 2 or 3 substituents, independently selected from hydroxy, C₁-C₆-alkyl, C₁-C₆-hydroxyalkyl, amino(C₁-C₆-alkyl), mono-(C₁-C₆-alkyl)amino(C₁-C₆-alkyl) and di-(C₁-C₆-alkyl)amino(C₁-C₆-alkyl),
to replace at least a part or all of the chlorine atoms by -X¹-R¹ groups.

A further object of the invention relates a modified polysaccharide, obtainable by the process described above and in the following.

A further object of the invention relates to the use of a a modified polysaccharide as defined above and in the following in
- as an intermediates that is susceptible for a further substitution, e.g. with polymerizable groups,
- as an auxiliary in pharmacy or cosmetics, preferably as or in coatings for solid drug forms,
- as surface-active compound,
- as or in (an) adhesive(s), or
- as or in (a) coating(s) for the textile, paper, printing and leather industry.

### DETAILED DESCRIPTION OF THE INVENTION

The present invention has the following advantages:
- The modified polysaccharides can provided with hydrophilic or hydrophobic properties within a wide range.
- The modified polysaccharides can act as intermediates that are susceptible for a further substitution, e.g. with polymerizable groups.
- The modified polysaccharides exhibit high affinity to various surfaces, in particular inorganic surfaces, such as metals, e.g. stainless steel, glass, ceramic, mineral materials, and polymer materials and are suitable for modifying the surface properties of these materials.
- The modified polysaccharides are based on nontoxic starting materials from renewable sources and are biodegradable.

In the context of the present invention, the expression "alkyl" comprises straight-chain or branched alkyl groups. Alkyl is preferably C₁-C₃₀-alkyl, more preferably C₁-C₂₀-alkyl and most preferably C₁-C₁₂-alkyl. Examples of alkyl groups are especially methyl, ethyl, n-propyl, isopropyl, n-butyl, isobutyl, sec-butyl, tert-butyl, n-pentyl, 1-methylbutyl, 1-ethylpropyl, neo-pentyl, n-hexyl, 1-methylpentyl, 2-methylpentyl, 1-ethylbutyl, 2-ethylbutyl, n-heptyl, 1-methylhexyl, 2-methylhexyl, 1-ethylpentyl, 1-propylbutyl, 2-ethylpentyl, n-octyl, 1-methylheptyl, 2-methylheptyl, 1-ethylhexyl, 2-ethylhexyl, 1-propylpentyl, 2-propylpentyl, n-nonyl, 1-methyloctyl, 2-methyloctyl, 1-ethylheptyl, 2-ethylheptyl, 1-propylhexyl, 2-propylhexyl, 1-butylpentyl, n-decyl, 2-methyldecyl, 1-methylnonyl, 2-methylnonyl, 1-ethyloctyl, 2-ethyloctyl, 1-propylheptyl, 2-propylheptyl, 1-butylhexyl, 2-butylhexyl, n-undecyl, 2-ethylnonyl, 1-propyloctyl, 2-propyloctyl, 1-butylheptyl, 2-butylheptyl, 1-pentylhexyl, n-dodecyl, 2-ethyldecyl, 2-propylnonyl,1-butyloctyl, 2-butyloctyl, 1-pentylheptyl, 2-pentylheptyl, 2-propyldecyl, n-tridecyl, 1-pentyloctyl, 2-pentyloctyl, 1-hexylheptyl, 2-butylnonyl, n-tetradecyl, 1-hexyloctyl, 2-hexyloctyl, 2-pentylnonyl, 2-hexylnonyl, 2-pentyldecyl, 2-butyldecyl,n-hexadecyl, 1-heptyloctyl, 2-heptylnonyl, 2-hexyldecyl, 2-heptyldecyl, n-octadecyl, 2-octyldecyl, n-eicosyl, 2-nonylundecyl, 2-octylundecyl, 2-heptylundecyl, 2-hexylundecyl, 2-pentylundecyl, 2-butylundecyl, 2-propylundecyl, 2-ethylundecyl, 2-methylundecyl, 2-decyldodecyl, 2-nonyldodecyl, 2-octyldodecyl, 2-heptyldodecyl, 2-hexyldodecyl, 2-pentyldodecyl, 2-butyldodecyl, 2-propyldodecyl, 2-ethyldodecyl, 2-methyldodecyl, 2-undecyltridecyl, 2-decyltridecyl, 2-nonyltridecyl, 2-octyltridecyl, 2-heptyltridecyl, 2-hexyltridecyl, 2-pentyltridecyl, 2-butyltridecyl, 2-propyltridecyl, 2-ethyltridecyl, 2-methyltridecyl, 2-undecyltetradecyl, 2-decyltetradecyl, 2-nonyltetradecyl, 2-octyltetradecyl, 2-hetyltetradecyl, 2-hexyltetradecyl, 2-pentyltetradecyl, 2-butyltetradecyl, 2-propyltetradecyl, 2-ethyltetradecyl, 2-methyltetradecyl, 2-tetradecylhexadecyl, 2-tridecylhexadecyl, 2-dodecylhexadecyl, 2-undecylhexadecyl, 2-decylhexadecyl, 2-nonylhexadecyl, 2-octylhexadecyl, 2-heptylhexadecyl, 2-hexylhexadecyl, 2-pentylhexadecyl, 2-butylhexadecyl, 2-propylhexadecyl, 2-ethylhexadecyl, 2-methylhexadecyl, 2-dodecyloctadecyl, 2-undecyloctadecyl, 2-decyloctadecyl, 2-nonyloctadecyl, 2-octyloctadecyl, 2-heptyloctadecyl, 2-hexyloctadecyl, 2-pentyloctadecyl, 2-butyloctadecyl, 2-propyloctadecyl, 2-ethyloctadecyl, 2-methyloctadecyl, 2-decyleicosanyl, 2-nonyleicosanyl, 2-octyleicosanyl, 2-heptyleicosanyl, 2-hexyleicosanyl, 2-pentyleicosanyl, 2-butyleicosanyl, 2-propyleicosanyl, 2-ethyleicosanyl, 2-methyleicosanyl, 2-octadecyldocosanyl, 2-heptadecyldocosanyl, 2-hexadecyldocosanyl, 2-pentadecyldocosanyl, 2-tetradecyldocosanyl, 2-tridecyldocosanyl, 2-undecyldocosanyl, 2-decyldocosanyl, 2-nonyldocosanyl, 2-octyldocosanyl, 2-heptyldocosanyl, 2-hexyldocosanyl, 2-pentyldocosanyl, 2-butyldocosanyl, 2-propyldocosanyl, 2-ethyldocosanyl, 2-methyldocosanyl, 2-docosanyltetracosanyl, 2-hexadecyltetracosanyl, 2-pentadecyltetracosanyl, 2-pentadecyltetracosanyl, 2-tetradecyltetracosanyl, 2-tridecyltetracosanyl, 2-dodecyltetracosanyl, 2-undecyltetracosanyl, 2-decyltetracosanyl, 2-nonyltetracosanyl, 2-octyltetracosanyl, 2-heptyltetracosanyl, 2-hexyltetracosanyl, 2-pentyltetracosanyl, 2-butyltetracosanyl, 2-propyltetracosanyl, 2-ethyltetracosanyl, 2-methyltetracosanyl, 2-dodecyloctacosanyl, 2-undecyloctacosanyl, 2-decyloctacosanyl, 2-nonyloctacosanyl, 2-octyloctacosanyl, 2-heptyloctacosanyl, 2-hexyloctacosanyl, 2-pentyloctacosanyl, 2-butyloctacosanyl, 2-propyloctacosanyl, 2-ethyloctacosanyl and 2-methyloctacosanyl.

A special embodiment of alkyl groups are short-chain C₁-C₆-alkyl groups. The C₁-C₆-alkyl groups are preferably selected from methyl, ethyl, propyl, isopropyl, n-butyl, 2-butyl, sec-butyl, tert-butyl, n-pentyl and n-hexyl.

A further special embodiment of alkyl groups are long-chain C₇-C₃₀-alkyl groups. The term C₇-C₃₀-alkyl includes straight-chain and branched C₇-C₃₀-alkyl groups. These are preferably straight-chain and branched C₉-C₂₅-alkyl, particularly preferably C₁₀-C₂₂-alkyl, and especially C₁₁-C₁₉-alkyl groups. Preference is given in this connection to predominantly linear alkyl radicals, as also occur in natural or synthetic fatty acids and fatty alcohols, and also oxo alcohols. These include, in particular, n-heptyl, n-octyl, 2-ethylhexyl, 1,1,3,3-tetramethylbutyl, n-nonyl, n-decyl, n-undecyl, n-dodecyl, n-tridecyl, myristyl, pentadecyl, palmityl (-cetyl), heptadecyl, octadecyl, nonadecyl, arachidyl, behenyl, lignocerenyl, cerotinyl, melissinyl, etc.

Examples of alkyl groups whose carbon chains are interrupted by one or more nonadjacent groups are especially 2-methoxyethyl, 2-ethoxyethyl, 2-propoxyethyl, 2-isopropoxyethyl, 2-butoxyethyl, 2- and 3-methoxypropyl, 2- and 3-ethoxypropyl, 2-and 3-propoxypropyl, 2- and 3-butoxypropyl, 2- and 4-methoxybutyl, 2- and 4-ethoxybutyl, 2- and 4-propoxybutyl, 3,6-dioxaheptyl, 3,6-dioxaoctyl, 4,8-dioxanonyl, 3,7-dioxaoctyl, 3,7-dioxanonyl, 4,7-dioxaoctyl, 4,7-dioxanonyl, 2- and 4-butoxybutyl, 4,8-dioxadecyl, 3,6,9-trioxadecyl, 3,6,9-trioxaundecyl, 3,6,9-trioxadodecyl, 3,6,9,12-tetraoxatridecyl and 3,6,9,12-tetraoxatetradecyl; 2-methylthioethyl, 2-ethylthioethyl, 2-propylthioethyl, 2-isopropylthioethyl, 2-butylthioethyl, 2- and 3-methylthiopropyl, 2- and 3-ethylthiopropyl, 2- and 3-propylthiopropyl, 2- and 3-butylthiopropyl, 2- and 4-methylthiobutyl, 2- and 4-ethylthiobutyl, 2- and 4-propylthiobutyl, 3,6-dithioheptyl, 3,6-dithiooctyl, 4,8-dithiononyl, 3,7-dithiooctyl, 3,7-dithiononyl, 2- and 4-butylthiobutyl, 4,8-dithiodecyl, 3,6,9-trithiodecyl, 3,6,9-trithioundecyl, 3,6,9-trithiododecyl, 3,6,9,12-tetrathiotridecyl and 3,6,9,12-tetrathiotetradecyl; 2-monomethyl- and 2-monoethylaminoethyl, 2-dimethylaminoethyl, 2- and 3-dimethylaminopropyl, 3-monoisopropylaminopropyl, 2-and 4-monopropylaminobutyl, 2-dimethylaminobutyl and 4-dimethylaminobutyl.

The above remarks regarding alkyl also apply to the alkyl moieties in C₂-C₃₀-hydroxyalkyl, amino(C₁-C₃₀-alkyl), mono-(C₁-C₆-alkyl)amino(C₁-C₃₀-alkyl) and di-(C₁-C₆-alkyl)amino(C₁-C₃₀-alkyl).

C₁-C₃₀-hydroxyalkyl and C₂-C₃₀-hydroxyalkyl is derived from the before mentioned C₁-C₃₀-alkyl radicals which contain one, two, three or four, especially one or two hydroxyl groups, more especially one hydroxyl group. A special embodiment are C₇-C₃₀-hydroxyalkyl groups derived from fatty alcohols of natural or technical origin. Examples of C₂-C₃₀-hydroxyalkyl groups are 2-hydroxyethyl, 2- and 3-hydroxypropyl, 1-hydroxyprop-2-yl, 3- and 4-hydroxybutyl, 1-hydroxybut-2-yl, 5-hydroxypentyl, 6-hydroxyhexyl, 7-hydroxyheptyl, 8-hydroxyoctyl, 8-hydroxy-4-oxaoctyl, 9-hydroxynonyl, 10-hydroxydecyl, 11-hydroxyheptadecyl, 8,9-bishydroxyheptadecyl and 3-hydroxyheptadecyl.

The term C₃-C₃₀-alkenyl stands for straight-chain and branched alkenyl groups which can be mono-, di- or polyunsaturated (depending on the chain length). Preference is given to C₇-C₃₀-alkenyl, more preferably C₉-C₂₅-alkenyl, in particular C₁₀-C₂₂-alkenyl. These include, in particular, octenyl, nonenyl, decenyl, undecenyl, dodecenyl, tridecenyl, tetradecenyl, pentadecenyl, hexadecenyl, heptadecenyl, octadecenyl, nonadecenyl, linolyl, linolenyl, elaostearyl, etc.

C₃-C₂₀-cycloalkyl denotes a mono-, bi- or tricyclic hydrocarbon radical having from 3 to 20, preferably 4 to 12, more preferably 5 to 8, carbon atoms. C₃-C₂₀-cycloalkyl is preferably cyclopropyl, cyclobutyl, cyclopentyl, cyclohexyl, cycloheptyl cyclooctyl, cyclododecyl, cyclopentadecyl, norbornyl, bicyclo[2.2.2]octyl or adamantyl.

Examples of substituted cycloalkyl groups are especially 2- and 3-methylcyclopentyl, 2- and 3-ethylcyclopentyl, 2-, 3- and 4-methylcyclohexyl, 2-, 3- and 4-ethylcyclohexyl, 2-, 3- and 4-propylcyclohexyl, 2-, 3- and 4-isopropylcyclohexyl, 2-, 3- and 4-butylcyclohexyl, 2-, 3- and 4-sec.-butylcyclohexyl, 2-, 3- and 4-tert-butylcyclohexyl, 2-, 3- and 4-methylcycloheptyl, 2-, 3- and 4-ethylcycloheptyl, 2-, 3- and 4-propyl-cycloheptyl, 2-, 3- and 4-isopropylcycloheptyl, 2-, 3- and 4-butylcycloheptyl, 2-, 3- and 4-sec-butylcycloheptyl, 2-, 3- and 4-tert-butylcycloheptyl, 2-, 3-, 4- and 5-methyl-cyclooctyl, 2-, 3-, 4- and 5-ethylcyclooctyl, 2-, 3-, 4- and 5-propylcyclooctyl, 2-, 3-, 4-and 5-hydroxycyclohexyl.

C₃-C₂₀-aryl comprises unsubstituted and substituted aryl groups and is preferably phenyl, tolyl, xylyl, mesityl, naphthyl, fluorenyl, anthracenyl, phenanthrenyl, naphthacenyl, more preferred phenyl, tolyl, xylyl or mesityl. In particular, aryl is phenyl.

Aryl which bears one or more radicals is, for example, 2-, 3- and 4-methylphenyl, 2,4-, 2,5-, 3,5- and 2,6-dimethylphenyl, 2,4,6-trimethylphenyl, 2-, 3- and 4-ethylphenyl, 2,4-, 2,5-, 3,5- and 2,6-diethylphenyl, 2,4,6-triethylphenyl, 2-, 3- and 4-propylphenyl, 2,4-, 2,5-, 3,5- and 2,6-dipropylphenyl, 2,4,6-tripropylphenyl, 2-, 3- and 4-isopropylphenyl, 2,4-, 2,5-, 3,5- and 2,6-diisopropylphenyl, 2,4,6-triisopropylphenyl, 2-, 3- and 4-butylphenyl, 2,4-, 2,5-, 3,5- and 2,6-dibutylphenyl, 2,4,6-tributylphenyl, 2-, 3- and 4-isobutylphenyl, 2,4-, 2,5-, 3,5- and 2,6-diisobutylphenyl, 2,4,6-triisobutylphenyl, 2-, 3-and 4-sec-butylphenyl, 2,4-, 2,5-, 3,5- and 2,6-di-sec-butylphenyl, 2,4,6-tri-sec-butylphenyl, 2-, 3- and 4-tert-butylphenyl, 2,4-, 2,5-, 3,5- and 2,6-di-tert-butylphenyl and 2,4,6-tri-tert-butylphenyl; 2-, 3- and 4-methoxyphenyl, 2,4-, 2,5-, 3,5- and 2,6-dimethoxyphenyl, 2,4,6-trimethoxyphenyl, 2-, 3- and 4-ethoxyphenyl, 2,4-, 2,5-, 3,5-and 2,6-diethoxyphenyl, 2,4,6-triethoxyphenyl, 2-, 3- and 4-propoxyphenyl, 2,4-, 2,5-, 3,5- and 2,6-dipropoxyphenyl, 2-, 3- and 4-isopropoxyphenyl, 2,4-, 2,5-, 3,5- and 2,6-diisopropoxyphenyl, 2-, 3- and 4-butoxyphenyl and 2-, 3- and 4-hydroxyphenyl.

### Step a)

Preferably, providing the chlorinated polysaccharide in step a) comprises
a1) dissolving a polysaccharide in a solvent system which comprises at least one ionic liquid, and
a2) reacting the polysaccharide with a chlorinating agent.

A preferred method for chlorinating polysaccharides is described in US 2011/0175023 A1. The teaching of this document is incorporated herein by reference.

In the context of the present invention, the expression "polysaccharide" also comprises carbohydrates which have a low number, e.g. two to six, monosaccharide units and are denoted elsewhere as "oligosaccharides".

Preferably, the polysaccharide employed in step a1) is selected from cellulose, hemicellulose, starch, glycogen, dextran, tunicin, inulin, chitin, alginic acid, chemically modified cellulose, etc. More preferably, the polysaccharide employed in step a1) is selected from cellulose, hemicellulose and chemically modified cellulose.

Glycogen is a multibranched polysaccharide that has a similar structure to amylopectin but is usually more extensively branched and compact than starch. Dextran is a complex, branched polysaccharide made of glucose molecules composed of chains of varying lengths. The straight chain of dextran consists of α-1,6 glycosidic linkages between the glucose units, whereas the branches begin from α-1,3 linkages. Tunicin is also a polysaccharide made of glucose units and is present in the mantle (or tunic) of the tunicates, a marine organism. Inulins are polymers composed mainly of fructose units and typically have a terminal glucose unit. The fructose units in inulins are joined by a β-2,1 glycosidic bond. Further examples of suitable polysaccharides are the polycondensates of D-fructose, e.g. inulin.

A natural derivative of glucose is chitin, a long-chain polymer of N-acetylglucosamine. The polysaccharides, in particular cellulose, may also be chemically modified, for example by etherification or esterification of at least a part of the of hydroxyl groups.

A special type hemicellulose is xyloglucan that occurs e.g. in the primary cell wall of all vascular plants. Xyloglucan has a backbone of β-1,4-linked glucose residues, most of which substituted with 1,6-linked xylose sidechains.

Preferably, the polysaccharide employed in step a1) comprises -CH₂OH groups. In polysaccharides with -CH₂OH groups (hydroxyl methylene groups), usually the chlorination of the hydroxyl groups of the -CH₂OH groups is preferred over the chlorination of the hydroxyl groups directly bound the ring carbon atoms of the polysaccharide.

Preferably, the polysaccharide employed in step a1) is selected from cellulose, hemicellulose and chemically modified cellulose. In a more preferred embodiment of the invention cellulose is used as polysaccharide. Most preferably the cellulose used is unmodified.

Preferably, the polysaccharides used for the process of the invention have a degree of polymerization (DP) of at least 10, more preferably of at least 50, in particular of at least 150, especially of at least 300. The maximum DP of the polysaccharides preferably does not exceed 2500, more preferably does not exceed 2000, in particular does not exceed 1800. The maximum DP of the polysaccharides, for example, does not exceed 1500, more preferably does not exceed 1000 or does not exceed 800.

Preferably, in the process of the invention a cellulose is employed that has a DP of at least 10, more preferably of at least 50, in particular of at least 150, especially of at least 300 least 50. The DP of the cellulose preferably does not exceed 2500, more preferably does not exceed 2000, in particular does not exceed 1800. The maximum DP of the cellulose, for example, does not exceed 1500, more preferably does not exceed 1000 or does not exceed 800.

The degree of polymerization (DP) is the number of repeat units in an average polymer chain. DP can be calculated as follows: DP = total Mw of the polymer/Mw of the repeating unit. The molecular weight is the weight average molecular weight. The DP can be measured e.g. by Gel Permeation Chromatography (GPC) or Size Exclusion Chromatography (SEC) A further well known method that is used as a standard for DP measurement in the pulp industry is the Cuprene method. According to this method a polysaccharide sample is dissolved in cupriethylenediamine hydroxide and the intrinsic viscosity of this solution is measured that allows the determination of the degree of polymerization.

The solvent system employed in step a1) may consist of one solvent or a mixture of solvents. The solvent system may consist of only one ionic liquid or a mixture of at least two different ionic liquids or a mixture of at least one ionic liquids and at least one other organic, non-ionic solvent.

Suitable organic solvents are non-ionic, polar solvents which can be mixed homogeneously with ionic liquids and do not lead to precipitation of the polysaccharide. Preferred organic solvents are ethers or ketons, for example dioxane, dimethyl sulfoxide, dimethylformamide, dimethylacetamide or sulfolane. Preferred is dioxane.

The content of ionic liquids in the solvent system employed in step a1) is preferably at least 20% by weight, more preferably at least 50% by weight and most preferably at least 80% or 90% by weight.

In one preferred embodiment of the invention the solvent system is a mixture comprising one or more ionic liquids and at least one non ionic solvent, in particular dioxane. In such mixture the content of ionic liquids is preferably from 20 to 90% by weight, the remainder being at least one organic non-ionic solvent.

The solvent system preferably contains no water or has only a low content of water of below 5% by weight, based on the total weight of the solvent system. In particular the content of water is below 2% by weight.

The term ionic liquid refers to salts (compounds composed of cations and anions) which at atmospheric pressure (1 bar) have a melting point of less than 200°C, preferably less than 150°C, particularly preferably less than 100°C and very particularly preferably less than 80°C.

Suitable ionic liquids that can be employed in step a) of the process of the invention are described in US 2011/0175023 A1, paragraphs [0028-0061]. This teaching is incorporated herein by reference.

Preferred cations are unsubstituted or substituted imidazolium ions. Particularly suitable imidazolium ions are 1-methylimidazolium, 1-ethylimidazolium, 1-(1-propyl)imidazolium, 1-(1-allyl)imidazolium, 1-(1-butyl)imidazolium, 1-(1-octyl)-imidazolium, 1-(1-dodecyl)imidazolium, 1-(1-tetradecyl)imidazolium, 1-(1-hexadecyl)-imidazolium, 1,3-dimethylimidazolium, 1,3-diethylimidazolium, 1-ethyl-3-methyl-imidazolium, 1-(1-butyl)-3-methylimidazolium, 1-(1-butyl)-3-ethylimidazolium, 1-(1-hexyl)-3-methylimidazolium, 1-(1-hexyl)-3-ethylimidazolium, 1-(1-hexyl)-3-butyl-imidazolium, 1-(1-octyl)-3-methylimidazolium, 1-(1-octyl)-3-ethylimidazolium, 1-(1-octyl)-3-butylimidazolium, 1-(1-dodecyl)-3-methylimidazolium, 1-(1-dodecyl)-3-ethyl-imidazolium, 1-(1-dodecyl)-3-butylimidazolium, 1-(1-dodecyl)-3-octylimidazolium, 1-(1-tetradecyl)-3-methylimidazolium, 1-(1-tetradecyl)-3-ethylimidazolium, 1-(1-tetradecyl)-3-butylimidazolium, 1-(1-tetradecyl)-3 octylimidazolium, 1-(1-hexadecyl)-3-methyl-imidazolium, 1-(1-hexadecyl)-3-ethylimidazolium, 1-(1-hexadecyl)-3-butylimidazolium, 1-(1-hexadecyl)-3-octylimidazolium, 1,2-dimethylimidazolium, 1,2,3 trimethyl-imidazolium, 1-ethyl-2,3-dimethylimidazolium, 1-(1-butyl)-2,3-dimethylimidazolium, 1-(1-hexyl)-2,3-dimethylimidazolium, 1-(1-octyl)-2,3-dimethylimidazolium, 1,4-dimethyl-imidazolium, 1,3,4-trimethylimidazolium, 1,4-dimethyl-3-ethylimidazolium, 3-methyl-imidazolium, 3-ethylimidazolium, 3-n-propylimidazolium, 3-n-butylimidazolium, 1,4-dimethyl-3-octylimidazolium, 1,4,5-trimethylimidazolium, 1,3,4,5-tetramethyl-imidazolium, 1,4,5-trimethyl-3-ethyhimidazolium, 1,4,5-trimethyl-3-butylimidazolium, 1,4,5-trimethyl-3-octylimidazolium, 1-prop-1-en-3-yl-3-methylimidazolium and 1-prop-1-en-3-yl-3-butylimidazolium. Especially suitable imidazolium ions (IVe) are 1,3-diethylimidazolium, 1-ethyl-3-methylimidazolium, 1-(n-butyl)-3-methylimidazolium.

Preferred cations are, in addition, pyridinium ions. These are selected, in particular, from pyridinium, 2-methylpyridinium, 2-ethylpyridinium, 5-ethyl-2-methylpyridinium and 2-methyl-3-ethylpyridinium and also 1-methylpyridinium, 1-ethylpyridinium, 1-(1-butyl)-pyridinium, 1-(1-hexyl)pyridinium, 1-(1-octyl)pyridinium, 1-(1-hexyl)pyridinium, 1-(1-octyl)pyridinium, 1-(1-dodecyl)pyridinium, 1-(1-tetradecyl)pyridinium, 1-(1-hexadecyl)-pyridinium, 1,2-dimethylpyridinium, 1-ethyl-2-methylpyridinium, 1-(1-butyl)-2-methyl-pyridinium, 1-(1-hexyl)-2-methylpyridinium, 1-(1-octyl)-2-methylpyridinium, 1-(1-dodecyl)-2-methylpyridinium, 1-(1-tetradecyl)-2-methylpyridinium, 1-(1-hexadecyl)-2-methylpyridinium, 1-methyl-2-ethylpyridinium, 1,2-diethylpyridinium, 1-(1-butyl)-2-ethyl-pyridinium, 1-(1-hexyl)-2-ethylpyridinium, 1-(1-octyl)-2-ethylpyridinium, 1-(1-dodecyl)-2-ethylpyridinium, 9-(1-tetradecyl)-2-ethylpyridinium, 1-(1-hexadecyl)-2-ethylpyridinium, 1,2-dimethyl-5-ethylpyridinium, 1,5-diethyl-2-methylpyridinium, 1-(1-butyl)-2-methyl-3-ethylpyridinium, 1-(1-hexyl)-2-methyl-3-ethylpyridinium and 1-(1-octyl)-2-methyl-3-ethylpyridinium, 1-(1-dodecyl)-2-methyl-3-ethylpyridinium, 1-(1-tetradecyl)-2-methyl-3-ethylpyridinium and 1-(1-hexadecyl)-2-methyl-3-ethylpyridinium.

Preferred cations are, in addition, unsubstituted or substituted pyrazolium ions. Particularly preferred pyrazolium ions are pyrazolium and 1,4-dimethylpyrazolium.

Preferred anions are chloride, bromide, hydrogensulfate, tetrachloroaluminate, thiocyanate, dicyanamide, methylsulfate, ethylsulfate, methanesulfonate, formate, acetate, dimethylphosphate, diethylphosphate, p-toluenesulfonate, tetrafluoroborate and hexafluorophosphate and methylmethylphosphonate (methylester of methylphosphonate).

Particularly preferred ionic liquids consist exclusively of an organic cation together with one of the anions mentioned. Most preferred are imdazolium salts with an imidazolium cation according to formula I and one of the above anions, specifically one of the particularly preferred anions, specifically acetate, chloride, dimethylphosphate or diethylphosphate or methylmethylphosphonate. Most preferred is acetate or chloride.

Suitable ionic liquids for use in the method according to the invention are commercially available, e.g. under the brand name Basionic® from BASF SE.

Advantageous compounds for use in the method according to the invention are, e.g.: 1-ethyl-3-methylimidazolium chloride (EMIM CI, Basionic ST 80), 1-ethyl-3-methylimidazolium methanesulfonate (EMIM CH3SO3, Basionic ST 35), 1-butyl-3-methylimidazolium chloride (BMIM CI, Basionic ST 70), 1-butyl-3-methylimidazolium methanesulfonate (BMIM CH3SO3, Basionic ST 78), methylimidazolium chloride (HMIM CI, Basionic AC 75), methylimidazolium hydrogensulfate (HMIM HSO4 Basionic AC 39), 1-ethyl-3-methylimidazolium hydrogensulfate (EMIM HSO4 Basionic AC 25), 1-butyl-3-methylimidazolium hydrogensulfate (BMIM HSO4 Basionic AC 28), 1-ethyl-3-methylimidazolium acetate (EMIM Acetate, Basionic BC 01), 1-butyl-3-methylimidazolium acetate (BMIM Acetate, Basionic BC 02).

Particular preference is given to 1-butyl-3-methyl¬imidazolium chloride, 1-ethyl-3-methylimidazolium chloride, 1-ethyl-3-methylimidazolium acetate, 1 butyl-3-methylimidazolium acetate and mixtures thereof.

In step a1) of the process of the invention, a solution of the polysaccharide, preferably cellulose, in the solvent system is prepared. The concentration of the polysaccharide is usually in the range from 0.1 to 50% by weight, based on the total weight of the solution, preferably from 0.2 to 40% by weight, particularly preferably from 0.3 to 30% by weight and very particularly preferably from 0.5 to 20% by weight.

This dissolution procedure can be carried out by known methods at room temperature or with heating, but above the melting point or softening temperature of the ionic liquid. If a solvent system comprising ionic liquids and non-ionic solvents is used, the polysaccharide may be dissolved in the ionic liquid first and the non-ionic solvent be added thereafter.

In step a2) of the process of the invention, the polysaccharides, preferably cellulose, is reacted with a chlorinating agent. The chlorinating agent may, for example, be added as such or in form of a solution in an appropriate solvent to the solution obtained after step a1).

Usual chlorinating agents may be used, for example thionyl chloride, methanesulfonyl chloride, chlorodimethyliminium chloride, phosphoryl chloride or para-toluenesulfonic chloride. A preferred chlorinating agent is thionyl chloride.

The chlorinating agent should be added at least in amounts to achieve the desired degree of substitution. The degree of substitution (DS) of polysaccharides is the average number of hydroxyl groups per six-ring unit of the polysaccharides substituted by a chloride. The degree of substitution (DS) of chlorinated cellulose is defined as the average number of substituted hydroxyl groups per anhydroglucose unit (AGU). The DS can be determined from the chlorine content detected in elemental analysis.

The chlorinated polysaccharide provided in step a) of the process of the invention preferably has a degree of substitution (DS) of at least 0.5, in particular of at least 1. Preferably, in the process of the invention a chlorinated polysaccharide obtained by the process of the afore-mentioned steps a1) and a2) is employed, having a degree of substitution (DS) of at least 0.5, in particular of at least 1.

Preferably, the chlorinated polysaccharide, provided in step a) comprises -CH₂-Cl groups. In particular, a chlorinated cellulose is provided in step a). The theoretical maximum of the DS in chlorinated cellulose is 3.0 (3 hydroxyl groups in the AGU). The first hydroxyl group in cellulose to be substituted by a chlorine atom will usually be the hydroxyl of the hydroxyl-methylene-group.

A preferred DS of the chlorinated cellulose provided in step a) is 0.5 to 3, more preferred is a DS of 0.8 to 3. Suitable chlorinated cellulose provided in step a) may have, for example a DS of 0.5 to 1.5 or from 0.8 to 1.5. According to the afore-mentioned process a DS of the chlorinated cellulose of at least 1.0 can be easily achieved.

The chlorinating agent may be added in excess, which means that more chlorinating agent may be added than required for the maximum DS. Non-reacted chlorinating agents may be removed by usual means, thionyl chloride may, for example, be removed by evaporation. The chlorinating agent, in particular thionyl chloride, does not only effect the substitution of the hydroxyl group by a chlorine atom but leads also to a degradation of the polysaccharides, in particular cellulose. Hence the chlorinated polysaccharides, for example chlorinated cellulose, preferably have a degree of polymerization (DP) which is lower less than the DP of the non-chlorinated polysaccharides. In particular the DP of the chlorinated polysaccharide provided in step a) of the process of the invention may be less than 90%, preferably less than 80%, more preferably less than 50%, and most preferably less than 20 or even less than 10% of the DP of the non chlorinated starting material.

Preferably, in step a) a chlorinated polysaccharide with a DP of less than 100, more preferably with a DP of 5 to 100, especially of 20 to 60, more especially of 30 to 50 is provided. In a special embodiment, in step a) a chlorinated polysaccharide with a DP of 10 to 20 is provided.

Preferably, in step a) a chlorinated cellulose with a DP of less than 100, more preferably with a DP of 5 to 100, especially of 20 to 60, more especially of 30 to 50 is provided. In a special embodiment, in step a) a chlorinated cellulose with a DP of 10 to 20 is provided.

In a preferred embodiment of the process of the invention, in step a) a chlorinated cellulose is provided that has a DS of 0.5 to 3, specifically of 0.5 to 1.5 and a DP of 10 to 100, specifically of 10 to 50. Most preferred is chlorinated cellulose with a DS of 0.5 to 1.5 and a DP of 5 to 100 or chlorinated cellulose of a DS of 0.8 to 1.5 and a DP of 10 to 50.

Preferably, in the chlorinated cellulose provided in step a) a high amount of the hydroxyl groups in the 6-position have been replaced by chlorine atoms. Especially, up to 99.9%, more especially, up to 100% of the hydroxyl groups in the 6-position have been replaced by chlorine atoms. In a preferred embodiment of the process of the invention, in step a) a chlorinated cellulose is provided, wherein 60 to 100%, preferably 80 to 100%, in particular 90 to 99.9% of the hydroxyl groups in the 6-position have been replaced by chlorine atoms.

In a preferred embodiment of the process of the invention, in step a) a chlorinated cellulose is provided, wherein 0 to 20%, more preferably 0.1 to 10% of the hydroxyl groups directly bound to the ring carbon atoms have been replaced by chlorine atoms. During the chlorinating reaction the reaction mixture is preferably kept at an elevated temperature; the temperature may be for example from 30 to 150°C, more preferably from 80 to 130°C. Preferably, the reaction in step a2) is performed at ambient pressure (1 bar). In general, the reaction in step a2) is carried out in air. However, it is also possible to carry it out under inert gas, i.e., for example, under N₂, a noble gas or a mixture thereof. Temperature and reaction time may be selected to achieve the desired degree of DS and DP. For the degradation no further additives like acids or nucleophiles (see WO 2007/101811, degradation by the use of acids or WO 2007/101813, degradation by nucleophiles) are required. Also the use of a base is not required. In a preferred embodiment the chlorination in step a2) is performed in absence of an additional base.

The reaction product obtained in step a2) comprises the solvent system on the basis of at least one ionic liquid and the chlorinated polysaccharides. The chlorinated polysaccharides may be isolated, if desired, by usual means. The chlorinated polysaccharides may for example be obtained from the reaction product by adding a coagulating solvent (non-solvent for chlorinated polysaccharides) or other coagulating agent, in particular a base or basic salt, for example ammonia or a solution comprising NH₄OH and separating the coagulated chlorinated polysaccharides from the solvent system. Depending on the way of mixing coagulating solvent and reaction mixture, different chlorinated species can be obtained. For example, predominantly monochlorinated species are obtained by pouring the coagulation solvent into the reaction mixture. On the other hand, predominantly di-chlorinated species are obtained by pouring the reaction mixture into the coagulation solvent.

### Step b)

In step b), the chlorinated polysaccharide is reacted with at least one compound of the formula (I)

HX¹-R¹ (I)

wherein
- X¹: is O or NR^{a}, where R^{a} is hydrogen, C₁-C₆-alkyl, C₃-C₂₀-cycloalkyl or C₆-C₂₀-aryl,
- R¹: is selected from C₁-C₃₀-alkyl, C₃-C₃₀-alkenyl, C₂-C₃₀-hydroxyalkyl, amino(C₁-C₃₀-alkyl), mono-(C₁-C₆-alkyl)amino(C₁-C₃₀-alkyl), di-(C₁-C₆-alkyl)amino(C₁-C₃₀-alkyl), C₃-C₈-cycloalkyl and C₆-C₂₀-aryl,
wherein the alkyl group of C₁-C₃₀-alkyl and C₂-C₃₀-hydroxyalkyl may be interrupted by one or more non-adjacent groups selected from -O-, -S-, -C(=O)- and -N(R^{b})-, where R^{b} is hydrogen, C₁-C₆-alkyl, C₃-C₂₀-cycloalkyl or C₆-C₂₀-aryl,
wherein the cycloalkyl and aryl groups in each case can be unsubstituted or substituted by 1, 2 or 3 substituents, independently selected from hydroxy, C₁-C₆-alkyl, C₁-C₆-hydroxyalkyl, amino(C₁-C₆-alkyl), mono-(C₁-C₆-alkyl)amino(C₁-C₆-alkyl) and di-(C₁-C₆-alkyl)amino(C₁-C₆-alkyl),
to replace at least a part or all of the chlorine atoms by -X¹-R¹ groups.

Preferably, the compound of the formula (I) is selected from
- compounds of the formula (I.1)

   HX¹¹-R¹¹ (I.1)

   wherein
   X¹¹ is O or NR^{a}, where R^{a} is hydrogen, C₁-C₆-alkyl, C₃-C₂₀-cycloalkyl or C₆-C₂₀-aryl,
   R¹¹ is selected from C₁-C₃₀-alkyl, C₃-C₂₀-cycloalkyl and C₆-C₂₀-aryl,
   wherein the cycloalkyl and aryl groups in each case can be unsubstituted or substituted by 1, 2 or 3 substituents, independently selected from hydroxy, C₁-C₆-alkyl, C₁-C₆-hydroxyalkyl, amino(C₁-C₆-alkyl), mono-(C₁-C₆-alkyl)amino(C₁-C₆-alkyl) and di-(C₁-C₆-alkyl)amino(C₁-C₆-alkyl),
- compounds of the formula (I.2)

   HX¹²-R¹² (I.2)

   wherein
   X¹² is O or NR^{a}, where R^{a} is hydrogen, C₁-C₆-alkyl, C₃-C₂₀-cycloalkyl or C₆-C₂₀-aryl,
   R¹² is selected C₂-C₃₀-hydroxyalkyl, amino(C₁-C₃₀-alkyl), mono-(C₁-C₆-alkyl)amino(C₁-C₃₀-alkyl) and di-(C₁-C₆-alkyl)amino(C₁-C₃₀-alkyl),
   wherein the cycloalkyl and aryl groups in each case can be unsubstituted or substituted by 1, 2 or 3 substituents, independently selected from hydroxy, C₁-C₆-alkyl, C₁-C₆-hydroxyalkyl, amino(C₁-C₆-alkyl), mono-(C₁-C₆-alkyl)amino(C₁-C₆-alkyl) and di-(C₁-C₆-alkyl)amino(C₁-C₆-alkyl),
- mixtures thereof.

Preferably, in the compounds of the formula (I), X¹ is O or NR^{a}, where R^{a} is hydrogen or C₁-C₄-alkyl.

Preferably, in the compounds of the formula (I.1), X¹¹ is O or NR^{a}, where R^{a} is hydrogen or C₁-C₄-alkyl.

Preferably, in the compounds of the formula (I.2), X¹² is O or NR^{a}, where R^{a} is hydrogen or C₁-C₄-alkyl.

Preferably, the compounds of the formula (I) and of the formula (I.1) are selected from alcanols, monoalkylamines, dialkylamines and mixtures thereof.

Preferably, the compounds of the formula (I) and of the formula (I.2) are selected from diols, amino alcohols, diamines and mixtures thereof.

In a preferred embodiment, the compounds (I) and (I.1) are selected from alkanols having 1 to 6 carbon atoms. In particular, the compounds (I) and (I.1) are selected from methanol, ethanol, n-propanol, isopropanol, n-butanol, tert.-butanol, n-pentanol, n-hexanol and mixtures thereof.

In a further preferred embodiment, the compounds (I) and (I.1) are selected from saturated and unsaturated alcohols having 7 to 30 carbon atoms. In particular, the compounds (I) and (I.1) are selected from enanthic alcohol (1-heptanol), capryl alcohol (1-octanol), 2-ethyl hexanol, pelargonic alcohol (1-nonanol), capric alcohol (1-decanol), undecyl alcohol (1-undecanol), lauryl alcohol (1-dodecanol), tridecyl alcohol (1-tridecanol), myristyl alcohol (1-tetradecanol), pentadecyl alcohol (1-pentadecanol), cetyl alcohol (1-hexadecanol), palmitoleyl alcohol (cis-9-hexadecen-1-ol), CH₃(CH₂)₅CH=CH(CH₂)₈OH, heptadecyl alcohol (1-n-heptadecanol), stearyl alcohol (1-octadecanol), isostearyl alcohol (16-methylheptadecan-1-ol), (CH₃)₂CH-(CH₂)₁₅OH, elaidyl alcohol (9E-octadecen-1-ol), CH₃(CH₂)₇CH=CH(CH₂)₈OH, oleyl alcohol (cis-9-octadecen-1-ol), linoleyl alcohol (9Z,12Z-octadecadien-1-ol), elaidolinoleyl alcohol (9E, 12E-octadecadien-1-ol), linolenyl alcohol (9Z, 12Z, 15Z-octadecatrien-1-ol), elaidolinolenyl alcohol (9E, 12E, 15-E-octadecatrien-1-ol), ricinoleyl alcohol (12-hydroxy-9-octadecen-1-ol), CH₃(CH₂)₅CH(OH)CH₂CH=CH(CH₂)₈OH, nonadecyl alcohol (1-nonadecanol), arachidyl alcohol (1-eicosanol), heneicosyl alcohol (1-heneicosanol), behenyl alcohol (1-docosanol), erucyl alcohol (cis-13-docosen-1-ol), CH₃(CH₂)₇CH=CH(CH₂)₁₂OH, lignoceryl alcohol (1-tetracosanol), ceryl alcohol (1-hexacosanol), 1-heptacosanol, montanyl alcohol, cluytyl alcohol (1-octacosanol), 1-nonacosanol, myricyl alcohol, melissyl alcohol (1-triacontanol).

Suitable compounds (I) and (I.1) which have exclusively a primary amino function are, for example, methylamine, ethylamine, n-propylamine, isopropylamine, n-butylamine, isobutylamine, sec-butylamine, tert-butylamine, pentylamine, hexylamine, cyclopentylamine, cyclohexylamine, aniline and benzylamine.

In a preferred embodiment, the compounds (I) and (I.1) are selected from monoalkylamines having 1 to 6 carbon atoms. In particular, the compounds (I) and (I.1) are selected from methylamine, ethylamine, n-propylamine, isopropylamine, n-butylamine, tert.-butylamine, n-pentylamine, n-hexylamine and mixtures thereof.

In a further preferred embodiment, the compounds (I) and (I.1) are selected from dialkylamines wherein both alkyl chains have 1 to 6 carbon atoms. In particular, the compounds (I) and (I.1) are selected from dimethylamine, diethylamine, methylethylamine, di-n-propylamine, diisopropylamine, diisobutylamine, di-sec-butylamine, di-tert-butylamine, dipentylamine, dihexylamine.

Suitable amines (I) are also dicyclopentylamine, dicyclohexylamine and diphenylamine.

In a further preferred embodiment, the compounds (I) and (I.1) are selected from monoalkylamines and dialkylamines, wherein at least one of the alkyl groups has 7 to 30 carbon atoms. In dialkylamines preferably one of the alkyl groups has 7 to 30 carbon atoms and the other has 1 to 4 alkyl groups. In particular, the compounds (I) and (I.1) are selected from heptylamine, octylamine, nonylamine, decylamine, undecylamine, dodecylamine, tridecylamine, tetradecylamine, pentadecylamine, hexadecylamine, heptadecylamine, octadecylamine, nonadecylamine, eicosylamine, heneicosylamine, docosylamine, tricosylamine, tetracosylamine, pentacosylamine, hexacosylamine, heptacosylamine, octacosylamine, nonacosylamine, triacontylamine. Suitable compounds (I) which have exclusively a primary amino function and in which the alkyl chain is interrupted by the heteroatom O are, for example, CH₃-O-C₂H₄-NH₂, C₂H₅-O-C₂H₄-NH₂, CH₃-O-C₃H₆-NH₂, C₂H₅-O-C₃H₆-NH₂, n-C₄H₉-O-C₄H₈-NH₂,

In a preferred embodiment, the compounds (I) and (I.2) are selected from diols having 2 to 30 carbon atoms, preferably 2 to 12 carbon atoms. In particular, the compounds (I) and (I.2) are selected from 1,2-ethanediol (ethylene glycol), 1,3-propanediol, 1,4-butanediol, 1,6-hexanediol, 1,5-pentanediol, 1,6-hexanediol, 1,7-heptanediol, 1,8-octanediol, 1,9-nonanediol, 1,10-decanediol, 1,11-undecanediol, 1,12-dodecanediol, 2-methyl-1,3-propanediol, 2,2-dimethyl-1,3-propanediol, diethylene glycol, triethylene glycol, tetraethylene glycol, pentaethylene glycol, hexaethylene glycol, neopentyl glycol, cyclohexanedimethylol and mixtures thereof. Particular preference is given to 1,2-ethanediol.

In a further preferred embodiment, the compounds (I) and (I.2) are selected from amino alcohols having 2 to 30 carbon atoms, preferably 2 to 12 carbon atoms, such as 2-aminoethanol, 2-(N-methylamino)ethanol, 3-aminopropanol, 4-aminobutanol, 1-ethylaminobutan-2-ol, 2-amino-2-methyl-1-propanol, 4-methyl-4-aminopentan-2-ol, N-methyldiethanolamine, etc. Particular preference is given to 2-aminoethanol.

In a further preferred embodiment, the compounds (I) and (I.2) are selected from diamines having 2 to 30 carbon atoms, preferably 2 to 12 carbon atoms. In particular, the compounds (I) and (I.2) are selected from ethylenediamine, n-propylenediamine, 1,4-butanediamine, 1,5-pentanediamine, 1,6-hexanediamine and mixtures thereof. Particular preference is given to ethylenediamine.

Suitable compounds of the formula (I) which have alkyl radicals interrupted by N are dialkylenetriamines, trialkylenetetramines and higher polyalkylenepolyamines. Preferred are Polyethyleneimines, consisting of from 2 to 15, preferably from 2 to 10, particularly preferably 2 or 3, ethyleneimine units. In particular, the compounds (I) are selected from 3-(dimethylamino)-n-propylamine, N,N-dimethylethylenediamine, N,N-diethylethylenediamine and N,N,N',N'-tetramethyldiethylenetriamine.

Preferably, in step b) the compound of the formula (I) is used in an at least 3-fold molar excess, preferably in an at least 6-fold molar excess, more preferably in an at least 10-fold molar excess over the chlorinated polysaccharide.

In a first preferred variant, the reaction in step b) is performed in the essential absence of extraneous solvent. The term "extraneous" denotes an additional (non-intrinsic) solvent, i.e. a component that is not a reactant or a reaction product. In the essential absence means that the amount of extraneous solvent used in step b) is less than 5% by weight, preferably less than 1 % by weight, based on the total weight of the reaction mixture.

In a second preferred embodiment, the reaction in step b) is performed in the presence of a polar solvent. Suitable polar solvents are selected from water, alcohols, such as methanol, ethanol, propanol, ketones, such as aceton and methylethylketon, ethers, such as tetrahydrofuran, diethylether, methyl-tert-butylether, esters, such as ethyl acetate, acetonitrile, ethylencarbonate, formamide, dimethylformamide (DMF), dimethylacetamide, dimethylsulfoxid (DMSO) and mixtures thereof. A preferred solvent for the reaction in step b) is ethanol. A further preferred solvent for the reaction in step b) is dimethylformamide, optionally in combination with water.

The reaction in step b) is preferably performed in the presence of a base. Suitable bases are, for example, alkali metal hydroxides, e.g. NaOH and KOH; alkali metal alkoxides, e.g. sodium methoxide, potassium methoxide, sodium tert-butoxide, potassium tert-butoxide, sodium tert-pentoxide and potassium tert-pentoxide; alkali metal hydrides, e.g. sodium hydride and potassium hydride; alkali metal carbonates, e.g. sodium carbonate and potassium carbonate, amines, e.g. triethylamine, diisopropylethylamine, etc.

In case of compound (I) being an amine or aminoalcohol, the compound (I) itself can act as base, if added in excess.

Preferably, the base is employed in at least stoichiometric amount with regard to the molar amount of the chlorine atoms of the chlorinated polysaccharide.

Preferably, in step b) the molar ratio of base to chlorine atoms of the chlorinated polysaccharide is in a range of from 1:1 to 10:1, more preferably in a range of from 1:1 to 2:1.

In a special embodiment, the reaction in step b) comprises a step-wise reaction of the chlorinated polysaccharide with at least two different compounds of the formula (I). In this embodiment, each step is performed in the presence of a substoichiometric amount of the base.

Preferably, the reaction product of step b) is isolated from the reaction discharge by precipitation.

The precipitation can comprise at least one of the following measures:
- reducing the temperature of the reaction discharge,
- removing a part of the volatile components of the reaction discharge,
- adding an anti-solvent to the reaction discharge.

The reduction of the temperature of the reaction discharge can be effected by known cooling methods.

Removing a part of the volatile components of the reaction discharge can be effected e.g. by know distillation methods.

In a preferred embodiment, the isolation of the reaction product of step b) comprises a precipitation with an anti-solvent (a solvent in which the product is insoluble). Suitable anti-solvents are preferably selected from among linear C₁-C₄-alkanols, acetone and mixtures thereof. The anti-solvent is particularly preferably selected from among methanol, ethanol, acetone and mixtures thereof.

The precipitated reaction product of step b) can be subjected to washing with a liquid washing medium after it has been separated off. Suitable washing media are those in which the reaction product of step b) does not dissolve or dissolves only in small amounts. Preferred washing media are the above-described anti-solvents used for the precipitation. A particularly preferred washing medium is methanol or acetone.

Preferably, the reaction product obtained in step b) is essentially free of chlorine atoms. Essentially free of chlorine means that the amount of chlorine atoms in the reaction product of step b) is less than 2% Cl as determined by elemental analysis.

A further object of the invention is a modified polysaccharide, obtainable by the process of the invention as defined before.

Preferably, the modified polysaccharides according to the invention or obtainable by the process of the invention have a degree of polymerization (DP) of 10 to 100, preferably 20 to 60, in particular 30 to 50. In a special embodiment, the modified polysaccharides have a DP of 10 to 20.

Preferably, the modified polysaccharides according to the invention or obtainable by the process of the invention have a DS of groups derived from the compounds of the formula (II) of from 0.02 to 0.1.

Preferably, the modified polysaccharides according to the invention or obtainable by the process of the invention have a DS of groups of the formula #-X-A-OH of from 0 to 0.99.

Preferably, the modified polysaccharides according to the invention or obtainable by the process of the invention are essentially free of chlorine atoms. Essentially free of chlorine means that the amount of chlorine atoms in the reaction product of step b) is less than 2% Cl as determined by elemental analysis.

### Personal care composition

The invention further provides a personal care composition comprising
A) at least modified polysaccharides according to the invention or obtainable by the process of the invention,
B) at least one cosmetically acceptable or pharmaceutically acceptable carrier.

Suitable personal care compositions are cosmetic compositions and hygiene compositions. With regard to suitable and preferred embodiments of the modified polysaccharides, reference is made to the general definition of the compounds (I), (I.1) and (I.2) as defined above.

The personal care compositions according to the invention preferably have a cosmetically or pharmaceutically acceptable carrier B) which is selected from
i) water,
ii) water-miscible organic solvents, preferably C₂-C₄-alkanols, in particular ethanol,
iii) oils, fats, waxes,
iv) esters of C₆-C₃₀-monocarboxylic acids with mono-, di- or trihydric alcohols different from iii),
v) saturated acyclic and cyclic hydrocarbons,
vi) fatty acids,
vii) fatty alcohols,
viii) propellant gases,
and mixtures thereof.

The compositions according to the invention have, for example, an oil or fat component B) which is selected from: hydrocarbons of low polarity, such as mineral oil; linear saturated hydrocarbons, preferably having more than 8 carbon atoms, such as tetradecane, hexadecane, octadecane etc.; cyclic hydrocarbons, such as decahydronaphthalene; branched hydrocarbons; animal and vegetable oils; waxes; wax esters; vaseline; esters, preferably esters of fatty acids, such as, for example, the esters of C₁-C₂₄-monoalcohols with C₁-C₂₂-monocarboxylic acids, such as isopropyl isostearate, n-propyl myristate, isopropyl myristate, n-propyl palmitate, isopropyl palmitate, hexacosanyl palmitate, octacosanyl palmitate, triacontanyl palmitate, dotriacontanyl palmitate, tetratriacontanyl palmitate, hexacosanyl stearate, octacosanyl stearate, triacontanyl stearate, dotriacontanyl stearate, tetratriacontanyl stearate; salicylates, such as C₁-C₁₀-salicylates, e.g. octyl salicylate; benzoate esters, such as C₁₀-C₁₅alkyl benzoates, benzyl benzoate; other cosmetic esters, such as fatty acid triglycerides, propylene glycol monolaurate, polyethylene glycol monolaurate, C₁₀-C₁₅-alkyl lactates, etc. and mixtures thereof.

Suitable silicone oils B) are, for example, linear polydimethylsiloxanes, poly(methylphenylsiloxanes), cyclic siloxanes and mixtures thereof. The number-average molecular weight of the polydimethylsiloxanes and poly(methylphenylsiloxanes) is preferably in a range from about 1000 to 150 000 g/mol. Preferred cyclic siloxanes have 4- to 8-membered rings. Suitable cyclic siloxanes are commercially available, for example, under the name cyclomethicone.

Preferred oil and fat components B) are selected from paraffin, isoparaffin, paraffin oils and isoparaffin oils; vaseline; natural fats and oils, such as castor oil, soya oil, peanut oil, olive oil, sunflower oil, sesame oil, avocado oil, cocoa butter, almond oil, peach kernel oil, castor oil, cod-liver oil, pig grease, spermaceti, spermaceti oil, sperm oil, wheatgerm oil, macadamia nut oil, evening primrose oil, jojoba oil; fatty alcohols, such as lauryl alcohol, myristyl alcohol, cetyl alcohol, stearyl alcohol, oleyl alcohol, cetyl alcohol; fatty acids, such as myristic acid, stearic acid, palmitic acid, oleic acid, linoleic acid, linolenic acid and saturated, unsaturated and substituted fatty acids different therefrom; waxes, such as beeswax, carnauba wax, candillila wax, spermaceti, and mixtures of the abovementioned oil and fat components.

Suitable cosmetically and pharmaceutically compatible oil and fat components B) are described in Karl-Heinz Schrader, Grundlagen und Rezepturen der Kosmetika [Fundamentals and formulations of cosmetics], 2nd edition, Verlag Hüthig, Heidelberg, pp. 319 - 355, to which reference is hereby made.

Suitable hydrophilic carriers B) are selected from water, mono-, di- or polyhydric alcohols having preferably 1 to 8 carbon atoms, such as ethanol, n-propanol, isopropanol, propylene glycol, glycerol, sorbitol, etc.

The cosmetic compositions according to the invention may be skin cosmetic, hair cosmetic, dermatological, hygiene or pharmaceutical compositions. The copolymers described above are particularly suitable as additives for hair and skin cosmetics.

The compositions according to the invention are preferably in the form of a gel, foam, spray, ointment, cream, emulsion, suspension, lotion, milk or paste. If desired, liposomes or microspheres can also be used.

The cosmetically or pharmaceutically active compositions according to the invention can additionally comprise cosmetically and/or dermatologically active ingredients and auxiliaries.

Preferably, the cosmetic compositions according to the invention comprise at least one modified polysaccharide A) as defined above, at least one carrier B) as defined above and at least one constituent different therefrom which is selected from cosmetically active ingredients, emulsifiers, surfactants, preservatives, perfume oils, thickeners, hair polymers, hair and skin conditioners, graft polymers, water-soluble or dispersible silicone-containing polymers, photoprotective agents, bleaches, gel formers, care agents, colorants, tints, tanning agents, dyes, pigments, consistency regulators, humectants, refatting agents, collagen, protein hydrolysates, lipids, antioxidants, antifoams, antistats, emollients and softeners.

Customary thickeners in formulations of this type are crosslinked polyacrylic acids and derivatives thereof, polysaccharides and derivatives thereof, such as xanthan gum, agar agar, alginates or tyloses, cellulose derivatives, e.g. carboxymethylcellulose or hydroxycarboxymethylcellulose, fatty alcohols, monoglycerides and fatty acids, polyvinyl alcohol and polyvinylpyrrolidone. Preference is given to using nonionic thickeners.

Suitable cosmetically and/or dermatologically active ingredients are, for example, coloring active ingredients, skin and hair pigmentation agents, tints, tanning agents, bleaches, keratin-hardening substances, antimicrobial active ingredients, photofilter active ingredients, repellent active ingredients, hyperemic substances, keratolytic and keratoplastic substances, antidandruff active ingredients, antiphlogistics, keratinizing substances, active ingredients which have an antioxidative effect or act as free-radical scavengers, skin-moisturizing or -humectant substances, refatting active ingredients, antierythamateous or antiallergic active ingredients and mixtures thereof.

Artificially skin-tanning active ingredients which are suitable for tanning the skin without natural or artificial irradiation with UV rays are, for example, dihydroxyacetone, alloxan and walnut shell extract. Suitable keratin-hardening substances are generally active ingredients, as are also used in antiperspirants, such as, for example, potassium aluminum sulfate, aluminum hydroxychloride, aluminum lactate, etc. Antimicrobial active ingredients are used in order to destroy microorganisms and to inhibit their growth and thus serve either as preservative or as deodorizing substance which reduces the formation or the intensity of body odor. These include, for example, customary preservatives known to the person skilled in the art, such as p-hydroxybenzoic acid esters, imidazolidinylurea, formaldehyde, sorbic acid, benzoic acid, salicylic acid, etc. Such deodorizing substances are, for example, zinc ricinoleate, triclosan, undecylenic acid alkyanolamides, triethyl citrate, chlorhexidine etc. Suitable photofilter active ingredients are substances which absorb UV rays in the UV-B and/or UB-A region. Suitable UV filters are, for example, 2,4,6-triaryl-1,3,5-triazines in which the aryl groups can each carry at least one substituent which is preferably selected from hydroxy, alkoxy, specifically methoxy, alkoxycarbonyl, specifically methoxycarbonyl and ethoxycarbonyl and mixtures thereof. Also suitable are p-aminobenzoic acid esters, cinnamic acid esters, benzophenones, camphor derivatives, and pigments which stop UV rays, such as titanium dioxide, talc and zinc oxide. Suitable repellent active ingredients are compounds which are able to keep away or drive away certain animals, in particular insects, from people. These include, for example, 2-ethyl-1,3-hexanediol, N,N-diethyl-m-toluamide etc. Suitable hyperemic substances, which stimulate circulation in the skin, are, for example, essential oils, such as pine, lavender, rosemary, juniper berry, horse chestnut extract, birch leaf extract, hay flower extract, ethyl acetate, camphor, menthol, peppermint oil, rosemary extract, eucalyptus oil, etc. Suitable keratolytic and keratoplastic substances are, for example, salicylic acid, calcium thioglycolate, thioglycolic acid and its salts, sulfur, etc. Suitable antidandruff active ingredients are, for example, sulfur, sulfur polyethylene glycol sorbitan monooleate, sulfur ricinol polyethoxylate, zinc pyrithione, aluminum pyrithione, etc. Suitable antiphlogistics, which counteract skin irritations, are, for example, allantoin, bisabolol, dragosantol, chamomile extract, panthenol, etc.

The compositions according to the invention can comprise, as active ingredient, e.g. as cosmetic and/or pharmaceutical active ingredient, at least one polymer which differs from the modified polysaccharides A) according to the invention. These include, quite generally, anionic, cationic, amphoteric and neutral polymers.

Examples of anionic polymers are homopolymers and copolymers of acrylic acid and methacrylic acid or salts thereof, copolymers of acrylic acid and acrylamide and salts thereof; sodium salts of polyhydroxycarboxylic acids, water-soluble or water-dispersible polyesters, polyurethanes, e.g. Luviset PUR® from BASF, and polyureas. Particularly suitable polymers are copolymers of t-butyl acrylate, ethyl acrylate, methacrylic acid (e.g. Luvimer® 100P), copolymers of ethyl acrylate and methacrylic acid (e.g. Luvimer® MAE), copolymers of N-tert-butylacrylamide, ethyl acrylate, acrylic acid (Ultrahold® 8, strong), copolymers of vinyl acetate, crotonic acid and, if appropriate, further vinyl esters (e.g. Luviset® grades), maleic anhydride copolymers, if appropriate reacted with alcohol, anionic polysiloxanes, e.g. carboxyfunctional, t-butyl acrylate, methacrylic acid (e.g. Luviskol® VBM), copolymers of acrylic acid and methacrylic acid with hydrophobic monomers, such as, for example, C₄-C₃₀-alkyl esters of (meth)acrylic acid, C₄-C₃₀-alkylvinyl esters, C₄-C₃₀-alkyl vinyl ethers and hyaluronic acid. Examples of anionic polymers are also vinyl acetate/crotonic acid copolymers, as are commercially available, for example, under the names Resyn® (National Starch) and Gafset® (GAF), and vinylpyrrolidone/vinyl acrylate copolymers obtainable, for example, under the trade name Luviflex® (BASF). Further suitable polymers are the vinylpyrrolidone/acrylate terpolymer available under the name Luviflex® VBM-35 (BASF) and sodium sulfonate-containing polyamides or sodium sulfonate-containing polyesters. Also suitable are vinylpyrrolidone/ethyl methacrylate/methacrylic acid copolymers, as are sold by Stepan under the names Stepanhold-Extra and - R1, and the Carboset® grades from BF Goodrich.

Suitable cationic polymers are, for example, cationic polymers with the INCI name Polyquaternium, e.g. copolymers of vinylpyrrolidone/N-vinylimidazolium salts (Luviquat® FC, Luviquat® HM, Luviquat® MS, Luviset Clear ®, Luviquat Supreme ®, Luviquat® Care), copolymers of N-vinylpyrrolidone/dimethylaminoethyl methacrylate, quaternized with diethyl sulfate (Luviquat® PQ 11), copolymers of N-vinylcaprolactam/N-vinylpyrrolidone/N-vinylimidazolium salts (Luviquat® Hold); cationic cellulose derivatives (polyquaternium-4 and -10), acrylamido copolymers (polyquaternium-7) and chitosan. Suitable cationic (quaternized) polymers are also Merquat® (polymer based on dimethyldiallylammonium chloride), Gafquat® (quaternary polymers which are formed by reacting polyvinylpyrrolidone with quaternary ammonium compounds), polymer JR (hydroxyethylcellulose with cationic groups) and plant-based cationic polymers, e.g. guar polymers, such as the Jaguar® grades from Rhodia. Also suitable are cationic polyurethanes, e.g. those described in WO 2006/069742.

Very particularly suitable polymers are neutral polymers, such as polyvinylpyrrolidones, copolymers of N-vinylpyrrolidone and vinyl acetate and/or vinylpropionate, polysiloxanes, polyvinylcaprolactam and other copolymers with N-vinylpyrrolidone, polyethyleneimines and salts thereof, polyvinylamines and salts thereof, cellulose derivatives, polyaspartic acid salts and derivatives. These include, for example, Luviflex® Swing (partially saponified copolymer of polyvinyl acetate and polyethylene glycol, BASF).

Suitable polymers are also nonionic, water-soluble or water-dispersible polymers or oligomers, such as polyvinylcaprolactam, e.g. Luviskol® Plus (BASF), or polyvinylpyrrolidone and copolymers thereof, in particular with vinyl esters, such as vinyl acetate, e.g. Luviskol® VA 37 (BASF); polyamides, e.g. based on itaconic acid and aliphatic diamines, as are described, for example, in DE-A-43 33 238.

Suitable polymers are also amphoteric or zwitterionic polymers, such as the octylacrylamide/methyl methacrylate/tert-butylaminoethyl methacrylate/2-hydroxypropyl methacrylate copolymers obtainable under the names Amphomer® (National Starch), and zwitterionic polymers, as are disclosed, for example, in the German patent applications DE 39 29 973, DE 21 50 557, DE 28 17 369 and DE 37 08 451. Acrylamidopropyltrimethylammonium chloride/acrylic acid or methacrylic acid copolymers and alkali metal and ammonium salts thereof are preferred zwitterionic polymers. Suitable zwitterionic polymers are also methacroylethylbetaine/methacrylate copolymers, which are commercially available under the name Amersette® (AMERCHOL), and copolymers of hydroxyethyl methacrylate, methyl methacrylate, N,N-dimethylaminoethyl methacrylate and acrylic acid (Jordapon®).

Suitable polymers are also nonionic, siloxane-containing, water-soluble or -dispersible polymers, e.g. polyether siloxanes, such as Tegopren® (Goldschmidt) or Belsil® (Wacker).

The formulation base of pharmaceutical compositions according to the invention preferably comprises pharmaceutically acceptable auxiliaries. The auxiliaries which are known for use in the field of pharmacy, food technology and related fields are pharmaceutically acceptable, in particular those listed in relevant pharmacopoeia (e.g. DAB Ph. Eur. BP NF), and other auxiliaries whose properties do not preclude a physiological application.

Suitable auxiliaries may be: glidants, wetting agents, emulsifying and suspending agents, preservatives, antioxidants, antiirritants, chelating agents, emulsion stabilizers, film formers, gelling agents, odor masking agents, resins, hydrocolloids, solvents, solubility promoters, neutralizing agents, permeation accelerators, pigments, quaternary ammonium compounds, refatting and superfatting agents, ointment, cream or oil base substances, silicone derivatives, stabilizers, sterilizing agents, propellants, drying agents, opacifiers, thickeners, waxes, softeners, white oils. One embodiment in this regard is based on expert knowledge in the art, as is presented, for example, in Fiedler, H. P. Lexikon der Hilfsstoffe für Pharmazie, Kosmetik und angrenzende Gebiete [Lexicon of auxiliaries for pharmacy, cosmetics and related fields], 4th edition, Aulendorf: ECV-Editio-Kantor-Verlag, 1996.

To produce the dermatological compositions according to the invention, the active ingredients can be mixed or diluted with a suitable auxiliary (excipient). Excipients can be solid, semisolid or liquid materials which can serve as vehicles, carriers or medium for the active ingredient. The admixing of further auxiliaries takes place, if desired, in the manner known to the person skilled in the art. Furthermore, the modified polysaccharides A) are suitable as auxiliaries in pharmacy, preferably as or in (a) coating(s) or binder(s) for solid drug forms. They can also be used in creams and as tablet coatings and tablet binders.

According to a preferred embodiment, the compositions according to the invention are a skin-cleansing composition.

Preferred skin-cleansing compositions are soaps of liquid to gel-like consistency, such as transparent soaps, luxury soaps, deodorant soaps, cream soaps, baby soaps, skin-protection soaps, abrasive soaps and syndets, pasty soaps, soft soaps and washing pastes, liquid washing, showering and bathing preparations, such as washing lotions, shower baths and gels, foam baths, oil baths and scrub preparations, shaving foams, lotions and creams.

According to a further preferred embodiment, the compositions according to the invention are cosmetic compositions for the care and protection of the skin, nail care compositions or preparations for decorative cosmetics.

Suitable skin cosmetic compositions are, for example, face tonics, face masks, deodorants and other cosmetic lotions. Compositions for use in decorative cosmetics comprise, for example, concealing sticks, stage make-up, mascara and eyeshadows, lipsticks, kohl pencils, eyeliners, blushers, powder and eyebrow pencils.

Furthermore, the copolymers according to the invention can be used in nose strips for pore cleansing, in antiacne compositions, repellents, shaving compositions, hair removal compositions, intimate care compositions, footcare compositions, and in babycare.

The skincare compositions according to the invention are, in particular, W/O or O/W skin creams, day and night creams, eye creams, face creams, antiwrinkle creams, moisturizing creams, bleaching creams, vitamin creams, skin lotions, care lotions and moisturizing lotions.

Skin cosmetic and dermatological compositions based on the copolymers described above exhibit advantageous effects. The polymers can, inter alia, contribute to the moisturization and conditioning of the skin and to the improvement in the skin feel. By adding the polymers according to the invention, in certain formulations it is also possible to achieve an improvement in skin compatibility.

Skin cosmetic and dermatological compositions preferably comprise at least one modified polysaccharide A) comprising at least one panthenol ester in copolymerized form, in an amount of from about 0.001 to 30% by weight, preferably 0.01 to 20% by weight, very particularly preferably 0.1 to 12% by weight, based on the total weight of the composition.

Depending on the field of use, the compositions according to the invention can be applied in a form suitable for skincare, such as, for example, as cream, foam, gel, stick, mousse, milk, spray (pump spray or propellant-containing spray) or lotion.

Besides the modified polysaccharides A) and suitable carriers, the skin cosmetic preparations can also comprise further active ingredients and auxiliaries customary in skin cosmetics, as described above. These include, preferably, emulsifiers, preservatives, perfume oils, cosmetic active ingredients, such as phytantriol, vitamin A, E and C, retinol, bisabolol, panthenol, photoprotective agents, bleaches, colorants, tinting agents, tanning agents, collagen, protein hydrolysates, stabilizers, pH regulators, dyes, salts, thickeners, gel formers, consistency regulators, silicones, humectants, refatting agents and further customary additives.

Preferred oil and fat components of these skin cosmetic and dermatological compositions are the abovementioned mineral and synthetic oils, such as, for example, paraffins, silicone oils and aliphatic hydrocarbons with more than 8 carbon atoms, animal and vegetable oils, such as, for example, sunflower oil, coconut oil, avocado oil, olive oil, lanolin, or waxes, fatty acids, fatty acid esters, such as, for example, triglycerides of C₆-C₃₀-fatty acids, wax esters, such as, for example, jojoba oil, fatty alcohols, vaseline, hydrogenated lanolin and acetylated lanolin, and mixtures thereof.

The modified polysaccharides A) according to the invention, comprising at least one panthenol ester in copolymerized form, can also be mixed with conventional polymers, if specific properties are to be established.

To establish certain properties, such as, for example, improving the feel to the touch, the spreading behavior, the water resistance and/or the binding of active ingredients and auxiliaries, such as pigments, the skin cosmetic and dermatological preparations can additionally also comprise conditioning substances based on silicone compounds. Suitable silicone compounds are, for example, polyalkylsiloxanes, polyarylsiloxanes, polyarylalkylsiloxanes, polyether siloxanes or silicone resins.

The cosmetic or dermatological preparations are produced by customary methods known to the person skilled in the art.

Preferably, the cosmetic and dermatological compositions are in the form of emulsions, in particular water-in-oil (W/O) or oil-in-water (O/W) emulsions. However, it is also possible to choose other types of formulation, for example hydrodispersions, gels, oils, oleogels, multiple emulsions, for example in the form of W/O/W or O/W/O emulsions, anhydrous ointments or ointment bases, etc.

Emulsions are produced by known methods. Besides at least one ampholytic copolymer, the emulsions usually comprise customary constituents, such as fatty alcohols, fatty acid esters and, in particular, fatty acid triglycerides, fatty acids, lanolin and derivatives thereof, natural or synthetic oils or waxes and emulsifiers in the presence of water. The selection of additives specific to the type of emulsion and the production of suitable emulsions is described, for example, in Schrader, Grundlagen und Rezepturen der Kosmetika [Fundamentals and formulations of cosmetics], Hüthig Buch Verlag, Heidelberg, 2nd edition, 1989, third part, to which reference is hereby expressly made.

A suitable emulsion, e.g. for a skin cream etc., generally comprises an aqueous phase which has been emulsified using a suitable emulsifier system in an oil or fat phase. A modified polysaccharide A) according to the invention can be used to provide the aqueous phase or the oil phase.

Preferred fatty components which may be present in the fat phase of the emulsions are: hydrocarbon oils, such as paraffin oil, purcellin oil, perhydrosqualene and solutions of microcrystalline waxes in these oils; animal and vegetable oils, such as sweet almond oil, avocado oil, calophylum oil, lanolin and derivatives thereof, castor oil, sesame oil, olive oil, jojoba oil, karite oil, hoplostethus oil; mineral oils whose distillation start under atmospheric pressure is at about 250°C and whose distillation end-point is at 410°C, such as, for example, vaseline oil; esters of saturated or unsaturated fatty acids, such as alkyl myristate, e.g. isopropyl, butyl or cetyl myristate, hexadecyl stearate, ethyl or isopropyl palmitate, octanoic or decanoic acid triglycerides and cetyl ricinoleate.

The fat phase can also comprise silicone oils soluble in other oils, such as dimethylpolysiloxane, methylphenylpolysiloxane and the silicone glycol copolymer, fatty acids and fatty alcohols.

It is also possible to use waxes, such as, for example, carnauba wax, candillila wax, beeswax, microcrystalline wax, ozokerite wax and Ca, Mg and Al oleates, myristates, linoleates and stearates.

Furthermore, an emulsion according to the invention can be in the form of a O/W emulsion. Such an emulsion usually comprises an oil phase, emulsifiers which stabilize the oil phase in the water phase, and an aqueous phase, which is usually present in thickened form. Suitable emulsifiers are preferably O/W emulsifiers, such as polyglycerol esters, sorbitan esters or partially esterified glycerides.

According to a further preferred embodiment, the compositions according to the invention are a shower gel, a shampoo formulation or a bath preparation.

Such formulations comprise at least one modified polysaccharide A) according to the invention and usually anionic surfactants as base surfactants and amphoteric and/or nonionic surfactants as cosurfactants. Further suitable active ingredients and/or auxiliaries are generally selected from lipids, perfume oils, dyes, organic acids, preservatives and antioxidants, and thickeners/gel formers, skin conditioners and humectants.

These formulations comprise preferably 2 to 50% by weight, preferably 5 to 40% by weight, particularly preferably 8 to 30% by weight, of surfactants, based on the total weight of the formulation.

All anionic, neutral, amphoteric or cationic surfactants usually used in body-cleansing compositions can be used in the washing, showering and bath preparations.

Suitable anionic surfactants are, for example, alkyl sulfates, alkyl ether sulfates, alkylsulfonates, alkylarylsulfonates, alkyl succinates, alkyl sulfosuccinates, N-alkoyl sarcosinates, acyl taurates, acyl isethionates, alkyl phosphates, alkyl ether phosphates, alkyl ether carboxylates, alpha-olefinsulfonates, in particular the alkali metal and alkaline earth metal salts, e.g. sodium, potassium, magnesium, calcium, and ammonium and triethanolamine salts. The alkyl ether sulfates, alkyl ether phosphates and alkyl ether carboxylates can have between 1 and 10 ethylene oxide or propylene oxide units, preferably 1 to 3 ethylene oxide units, in the molecule.

These include, for example, sodium lauryl sulfate, ammonium lauryl sulfate, sodium lauryl ether sulfate, ammonium lauryl ether sulfate, sodium lauryl sarcosinate, sodium oleyl succinate, ammonium lauryl sulfosuccinate, sodium dodecylbenzenesulfonate, triethanolamine dodecylbenzenesulfonate.

Suitable amphoteric surfactants are, for example, alkylbetaines, alkylamidopropyl-betaines, alkylsulfobetaines, alkyl glycinates, alkyl carboxyglycinates, alkyl amphoacetates or amphopropionates, alkyl amphodiacetates or amphodipropionates.

For example, cocodimethylsulfopropylbetaine, laurylbetaine, cocamidopropylbetaine or sodium cocamphopropionate can be used.

Suitable nonionic surfactants are, for example, the reaction products of aliphatic alcohols or alkylphenols having 6 to 20 carbon atoms in the alkyl chain, which may be linear or branched, with ethylene oxide and/propylene oxide. The amount of alkylene oxide is about 6 to 60 moles per mole of alcohol. Also suitable are alkylamine oxides, mono- or dialkylalkanolamides, fatty acid esters of polyethylene glycols, ethoxylated fatty acid amides, alkyl polyglycosides or sorbitan ether esters.

Furthermore, the washing, showering and bath preparations can comprise customary cationic surfactants, such as, for example, quaternary ammonium compounds, for example cetyltrimethylammonium chloride.

In addition, the shower gel/shampoo formulations can comprise thickeners, such as, for example, sodium chloride, PEG-55, propylene glycol oleate, PEG-120 methylglucose dioleate and others, and preservatives, further active ingredients and auxiliaries and water.

According to a particularly preferred embodiment, the compositions according to the invention are a hair treatment composition.

Hair treatment compositions according to the invention preferably comprise at least one modified polysaccharide A) in an amount in the range from about 0.1 to 30% by weight, preferably 0.5 to 20% by weight, based on the total weight of the composition.

The hair treatment compositions are preferably in the form of a hair spray, setting foam, hair mousse, hair gel, shampoo, hair foam, end fluid, neutralizer for permanent waving, hair colorant and bleach or hot-oil treatment. Depending on the field of use, the hair cosmetic preparations can be applied as (aerosol) spray, (aerosol) foam, gel, gel spray, cream, lotion or wax. Hair sprays here comprise both aerosol sprays and pump sprays without propellant gas. Hair foams comprise both aerosol foams and pump foams without propellant gas. Hair sprays and hair foams comprise preferably predominantly or exclusively water-soluble or water-dispersible components. If the compounds used in the hair sprays and hair foams according to the invention are water-dispersible, they can be used in the form of aqueous microdispersions with particle diameters of usually 1 to 350 nm, preferably 1 to 250 nm. The solids contents of these preparations are here usually in a range from about 0.5 to 20% by weight. These microdispersions generally require no emulsifiers or surfactants for their stabilization.

The hair cosmetic formulations according to the invention comprise, in a preferred embodiment,
a) 0.05 to 20% by weight of at least one modified polysaccharide A) according to the invention, comprising at least one panthenol ester in copolymerized form, as defined above,
b) 20 to 99.95% by weight of water and/or alcohol,
c) 0 to 50% by weight of at least one propellant gas,
d) 0 to 5% by weight of at least one emulsifier,
e) 0 to 3% by weight of at least one thickener, and
f) up to 25% by weight of further constituents.

Alcohol is to be understood as meaning all alcohols customary in cosmetics, e.g. ethanol, isopropanol, N-propanol.

Further constituents are to be understood as meaning the additives customary in cosmetics, for example propellants, antifoams, interface-active compounds, i.e. surfactants, emulsifiers, foam formers and solubilizers. The interface-active compounds used may be anionic, cationic, amphoteric or neutral. Further customary constituents may also be, for example, preservatives, perfume oils, opacifiers, active ingredients, UV filters, care substances, such as panthenol, collagen, vitamins, protein hydrolysates, alpha- and beta-hydroxycarboxylic acids, protein hydrolysates, stabilizers, pH regulators, dyes, viscosity regulators, gel formers, dyes, salts, humectants, refatting agents, complexing agents and further customary additives.

These also include all styling and conditioner polymers known in cosmetics which can be used in combination with the polymers according to the invention if very specific properties are to be established.

To establish certain properties, the preparations can additionally also comprise conditioning substances based on silicone compounds. Suitable silicone compounds are, for example, polyalkylsiloxanes, polyarylsiloxanes, polyarylalkylsiloxanes, polyether siloxanes, silicone resins or dimethicone copolyols (CTFA) and aminofunctional silicone compounds, such as amodimethicones (CTFA).

The ampholytic copolymers and polyelectrolyte complexes according to the invention are particularly suitable as setting agents in hairstyling preparations, in particular hair sprays (aerosol sprays and pump sprays without propellant gas) and hair foams (aerosol foams and pump foams without propellant gas).

In a preferred embodiment, spray preparations comprise
a) 0.1 to 10% by weight of at least one modified polysaccharide A), as defined above,
b) 20 to 99.9% by weight of water and/or alcohol,
c) 0 to 70% by weight of at least one propellant,
d) 0 to 20% by weight of further constituents.

Propellants are the propellants used customarily for hair sprays and aerosol foams. Preference is given to mixtures of propane/butane, pentane, dimethyl ether, 1,1-difluoroethane (HFC-152 a), carbon dioxide, nitrogen or compressed air.

A formulation preferred according to the invention for aerosol hair foams comprises
a) 0.1 to 10% by weight of at least one modified polysaccharide A) according to the invention, as defined above,
b) 55 to 99.8% by weight of water and/or alcohol,
c) 5 to 20% by weight of a propellant,
d) 0.1 to 5% by weight of an emulsifier,
e) 0 to 10% by weight of further constituents.

Emulsifiers which can be used are all emulsifiers customarily used in hair foams. Suitable emulsifiers may be nonionic, cationic or anionic or amphoteric.

Examples of nonionic emulsifiers (INCI nomenclature) are laureths, e.g. laureth-4; cetheths, e.g. cetheth-1, polyethylene glycol cetyl ether; cetheareths, e.g. cetheareth-25, polyglycol fatty acid glycerides, hydroxylated lecithin, lactyl esters of fatty acids, alkyl polyglycosides.

Examples of cationic emulsifiers are cetyldimethyl-2-hydroxyethylammonium dihydrogenphosphate, cetyltrimonium chloride, cetyltrimonium bromide, cocotrimonium methylsulfate, quaternium-1 to x (INCI).

Anionic emulsifiers can be selected, for example, from the group of alkyl sulfates, alkyl ether sulfates, alkylsulfonates, alkylarylsulfonates, alkyl succinates, alkyl sulfosuccinates, N-alkoyl sarcosinates, acyl taurates, acyl isethionates, alkyl phosphates, alkyl ether phosphates, alkyl ether carboxylates, alpha-olefinsulfonates, in particular the alkali metal and alkaline earth metal salts, e.g. sodium, potassium, magnesium, calcium, and ammonium and triethanolamine salts. The alkyl ether sulfates, alkyl ether phosphates and alkyl ether carboxylates can have between 1 and 10 ethylene oxide or propylene oxide units, preferably 1 to 3 ethylene oxide units, in the molecule.

A preparation suitable according to the invention for styling gels can, for example, have the following composition:
a) 0.1 to 10% by weight of at least one modified polysaccharide A) according to the invention, as defined above,
b) 80 to 99.85% by weight of water and/or alcohol,
c) 0 to 3% by weight, preferably 0.05 to 2% by weight, of a gel former,
d) 0 to 20% by weight of further constituents.

Gel formers which can be used are all gel formers customary in cosmetics. These include slightly crosslinked polyacrylic acid, for example carbomer (INCI), cellulose derivatives, e.g. hydroxypropylcellulose, hydroxyethylcellulose, cationically modified celluloses, polysaccharides, e.g. xanthan gum, caprylic/capric triglyceride, sodium acrylate copolymers, polyquaternium-32 (and) paraffinum liquidum (INCI), sodium acrylate copolymers (and) paraffinum liquidum (and) PPG-1 trideceth-6, acrylamidopropyltrimonium chloride/acrylamide copolymers, steareth-10 allyl ether acrylate copolymers, polyquaternium-37 (and) paraffinum liquidum (and) PPG-1 trideceth-6, polyquaternium 37 (and) propylene glycol dicaprate dicaprylate (and) PPG-1 trideceth-6, polyquaternium-7, polyquaternium-44.

The modified polysaccharides A) according to the invention can be used in cosmetic preparations as conditioners.

The modified polysaccharides A) according to the invention, as defined above, can preferably be used in shampoo formulations as setting agents and/or conditioners. Preferred shampoo formulations comprise
a) 0.05 to 10% by weight of at least one modified polysaccharide A) as defined above,
b) 25 to 94.95% by weight of water,
c) 5 to 50% by weight of surfactant,
c) 0 to 5% by weight of a further conditioner,
d) 0 to 10% by weight of further cosmetic constituents.

In the shampoo formulations, it is possible to use all anionic, neutral, amphoteric or cationic surfactants customarily used in shampoos.

Suitable anionic surfactants are, for example, alkyl sulfates, alkyl ether sulfates, alkylsulfonates, alkylarylsulfonates, alkyl succinates, alkyl sulfosuccinates, N-alkoyl sarcosinates, acyl taurates, acyl isethionates, alkyl phosphates, alkyl ether phosphates, alkyl ether carboxylates, alpha-olefinsulfonates, in particular the alkali metal and alkaline earth metal salts, e.g. sodium, potassium, magnesium, calcium, and ammonium and triethanolamine salts. The alkyl ether sulfates, alkyl ether phosphates and alkyl ether carboxylates can have between 1 and 10 ethylene oxide or propylene oxide units, preferably 1 to 3 ethylene oxide units, in the molecule.

Of suitability are, for example, sodium lauryl sulfate, ammonium lauryl sulfate, sodium lauryl ether sulfate, ammonium lauryl ether sulfate, sodium lauroyl sarcosinate, sodium oleyl succinate, ammonium lauryl sulfosuccinate, sodium dodecylbenzenesulfonate, triethanolamine dodecylbenzenesulfonate.

Suitable amphoteric surfactants are, for example, alkylbetaines, alkylamidopropylbetaines, alkylsulfobetaines, alkyl glycinates, alkyl carboxyglycinates, alkyl amphoacetates or amphopropionates, alkyl amphodiacetates or amphodipropionates.

For example, cocodimethylsulfopropylbetaine, laurylbetaine, cocamidopropylbetaine or sodium cocamphopropionate can be used.

Suitable nonionic surfactants are, for example, the reaction products of aliphatic alcohols or alkylphenols having 6 to 20 carbon atoms in the alkyl chain, which may be linear or branched, with ethylene oxide and/or propylene oxide. The amount of alkylene oxide is about 6 to 60 moles per mole of alcohol. Also suitable are alkylamine oxides, mono- or dialkylalkanolamides, fatty acid esters of polyethylene glycols, alkyl polyglycosides or sorbitan ether esters.

Furthermore, the shampoo formulations can comprise customary cationic surfactants, such as, for example, quaternary ammonium compounds, for example cetyltrimethylammonium chloride.

### Pharmaceutical composition

A further aspect of the invention is a pharmaceutical composition comprising
A) at least modified polysaccharides according to the invention or obtainable by the process of the invention,
B) optionally at least one pharmaceutically acceptable active ingredient, and
C) optionally at least one pharmaceutically acceptable excipient.

Preferably, the pharmaceutical composition comprises the component A) in a fraction of from about 0.001 to 50% by weight, particularly preferably 0.01 to 30% by weight, in particular 0.1 to 20% by weight, based on the total weight of the composition.

The pharmaceutical composition of the invention is suitable for administering in principle any type of active pharmaceutical ingredient B). These include benzodiazepines, antihypertensives, vitamins, cytostatics, in particular taxol, anesthetics, neuroleptics, antidepressants, antibiotics, antimycotics, fungicides, chemotherapeutics, urologics, thrombocyte aggregation inhibitors, sulfonamides, spasmolytics, hormones, immunoglobulins, sera, thyroid therapeutic agents, psychopharmacological agents, antiparkinsonians and other antihyperkinetic agents, ophthalmics, neuropathy preparations, calcium metabolism regulators, muscle relaxants, narcotics, antilipemics, hepatic therapeutic agents, coronary agents, cardiacs, immunotherapeutics, regulatory peptides and their inhibitors, hypnotics, sedatives, gynecological agents, antigouts, fibrinolytic agents, enzyme preparations and transport proteins, enzyme inhibitors, emetics, circulation-promoting agents, diuretics, diagnostics, corticoids, cholinergics, bile duct therapeutics, antiasthmatics, broncholytics, beta-receptor blockers, calcium antagonists, ACE inhibitors, antiarteriosclerotics, antiinflammatories, anticoagulants, antihypotensives, antihypoglycemics, antihypertonics, antifibrinolytics, antiepileptics, antiemetics, antidotes, antidiabetics, antiarrhythmics, antianemics, antiallergics, anthelmintics, analgesics, analeptics, aldosterone antagonists and slimming agents

Examples of suitable active ingredients B) are: acarbose, non-steroidal antirheumatics, cardiac glycosides, acetylsalicylic acid, virustatics, aclarubicin, acyclovir, cisplatin, actinomycin, alpha- and beta-sympathomimetics, allopurinol, alosetron, alprostadil, prostaglandins, amantadine, ambroxol, amlodipine, methotrexate, 5-aminosalicylic acid, amitriptyline, amlodipine, amoxicillin, anastrozole, atenolol, atorvastatin, azathioprine, balsalazide, beclomethasone, betahistine, bezafibrate, bicalutamide, diazepam and diazepam derivatives, budesonide, bufexamac, buprenorphine, methadone, calcium salts, potassium salts, magnesium salts, candesartan, carbamazepine, captopril, cefalosporins, celetoxib, cetirizine, chenodeoxycholic acid, ursodeoxycholic acid, theophylline and theophylline derivatives, trypsins, cimetidine, clarithromycin, clavulanic acid, clindamycin, clobutinol, clonidine, cotrimoxazole, codeine, caffeine, vitamin D and derivatives of vitamin D, colestyramine, cromoglicic acid, coumarin and coumarin derivatives, cysteine, cytarabine, cyclophosphamide, ciclosporin, cyproterone, cytarabine, dapiprazole, desogestrel, desonide, dihydralazine, diltiazem, ergot alkaloids, dimenhydrinate, dimethyl sulfoxide, dimethicone, dipyridamole, domperidone and domperidone derivatives, donepzil, dopamine, doxazosin, doxorubicin, doxylamine, dapiprazole, benzodiazepines, diclofenac, glycoside antibiotics, desipramine, econazole, ACE inhibitors, enalapril, ephedrine, epinephrine, epoetin and epoetin derivatives, morphinans, calcium antagonists, irinotecan, modafinil, orlistat, peptide antibiotics, phenytoin, riluzoles, risedronate, sildenafil, topiramate, macrolide antibiotics, esomeprazole, estrogen and estrogen derivatives, progestogen and progestogen derivatives, testosterone and testosterone derivatives, androgen and androgen derivatives, ethenzamide, etofenamate, etofibrate, fenofibrate, etofylline, etoposide, famciclovir, famotidine, felodipine, fenofibrate, fentanyl, fenticonazole, gyrase inhibitor, fluconazole, fludarabine, flunarizine, fluorouracil, fluoxetine, flurbiprofen, ibuprofen, flutamide, fluvastatin, follitropin, formoterol, fosfomicin, furosemide, fusidic acid, galantamine, gallopamil, ganciclovir, gemfibrozil, gentamicin, ginkgo, St John's wort, glibenclamide, urea derivatives as oral antidiabetics, glucagon, glucosamine and glucosamine derivatives, glutathione, glycerol and glycerol derivatives, hypothalamus hormones, goserelin, guanethidine, halofantrine, haloperidol, heparin and heparin derivatives, hyaluronic acid, hydralazine, hydrochlorothiazide and hydrochlorothiazide derivatives, salicylates, hydroxyzine, idarubicin, ifosfamide, imipramine, indometacin, indoramine, insulin, interferons, iodine and iodine derivatives, isoconazole, isoprenaline, glucitol and glucitol derivatives, itraconazole, ketoconazole, ketoprofen, ketotifen, lacidipine, lansoprazole, levodopa, levomethadone, thyroid hormones, lipoic acid and lipoic acid derivatives, lisinopril, lisuride, lofepramine, lomustine, loperamide, loratadine, maprotiline, mebendazole, mebeverine, meclozine, mefenamic acid, mefloquine, meloxicam, mepindolol, meprobamate, meropenem, mesalazine, mesuximide, metamizole, metformin, methotrexate, methylphenidate, methylprednisolone, metixen, metoclopramide, metoprolol, metronidazole, mianserin, miconazole, minocycline, minoxidil, misoprostol, mitomycin, mizolastine, moexipril, morphine and morphine derivatives, evening primrose, nalbuphine, naloxone, tilidine, naproxen, narcotine, natamycin, neostigmine, nicergoline, nicethamide, nifedipine, niflumic acid, nimodipine, nimorazole, nimustine, nisoldipine, adrenaline, and adrenaline derivatives, norfloxacin, novaminsulfone, noscapine, nystatin, ofloxacin, olanzapine, olsalazine, omeprazole, omoconazole, ondansetron, orlistat, oseltamivir, oxaceprol, oxacillin, oxiconazole, oxymetazoline, pantoprazole, paracetamol, paroxetine, penciclovir, oral penicillins, pentazocine, pentifylline, pentoxifylline, perphenazine, pethidine, plant extracts, phenazone, pheniramine, barbituric acid derivatives, phenylbutazone, phenytoin, pimozide, pindolol, piperazine, piracetam, pirenzepine, piribedil, piroxicam, pramipexol, pravastatin, prazosin, procaine, promazine, propiverine, propranolol, propyphenazone, prostaglandins, protionamide, proxyphylline, quetiapine, quinapril, quinaprilate, ramipril, ranitidine, reproterol, reserpine, ribavirin, rifampicin, risperidone, ritonavir, ropinirol, rosiglitazone, roxatidine, roxithromycin, ruscogenin, rutoside and rutoside derivatives, sabadilla, salbutamol, salmeterol, scopolamine, selegiline, sertaconazole, sertindole, sertraline, silicates, simvastatin, sitosterol, sotalol, spaglumic acid, sparfloxacin, spectinomycin, spiramycin, spirapril, spironolactone, stavudine, streptomycin, sucralfate, sufentanil, sulbactam, sulfonamides, sulfasalazine, sulpiride, sultamicillin, sultiam, sumatriptan, suxamethonium chloride, tacrine, tacrolimus, taliolol, tamoxifen, taurolidine, tazarotene, tegaserod, temazepam, teniposide, tenoxicam, terazosin, terbinafine, terbutaline, terfenadine, terlipressin, tertatolol, tetracyclines, tetryzoline, theobromine, theophylline, butizine, thiamazole, phenothiazines, thiotepa, tiagabine, tiapride, propionic acid derivatives, ticlopidine, timolol, tinidazole, tioconazole, tioguanine, tioxolone, tiropramide, tizanidine, tolazoline, tolbutamide, tolcapone, tolnaftate, tolperisone, topotecan, torasemide, antiestrogens, tramadol, tramazoline, trandolapril, tranylcypromine, trapidil, trazodone, triamcinolone and triamcinolone derivatives, triamterene, trifluperidol, trifluridine, trimethoprim, trimipramine, tripelennamine, triprolidine, trifosfamide, tromantadine, trometamol, tropalpin, troxerutin, tulobuterol, tyramine, tyrothricin, urapidil, ursodeoxycholic acid, chenodeoxycholic acid, valaciclovir, valdecoxib, valproic acid, vancomycin, vecuronium chloride, venlafaxine, verapamil, vidarabine, vigabatrine, viloxazine, vinblastine, vincamine, vincristine, vindesine, vinorelbine, vinpocetine, viquidil, warfarin, xantinol nicotinate, xipamide, zafirlukast, zalcitabine, zanamivir, zidovudine, zolmitriptan, zolpidem, zopiclone, zotepine and the like.

The active ingredients can, if desired, also be used in the form of their pharmaceutically acceptable salts or derivatives, and in the case of chiral active ingredients it is possible to employ both optically active isomers and racemates or mixtures of diastereoisomers. The compositions of the invention can, if desired, also comprise two or more active pharmaceutical ingredients.

The formulation base of pharmaceutical compositions of the invention preferably comprises pharmaceutically acceptable excipients C). Pharmaceutically acceptable excipients are those known to be usable in the area of pharmacy, food technology and adjacent sectors, in particular the excipients listed in relevant pharmacopeias (e.g. DAB, Ph. Eur., BP, USP, JP) and others, whose properties do not stand in the way of physiological use.

Suitable excipients C) may be: lubricants, wetting agents, emulsifying and suspending agents, antioxidants, anti-irritants, chelating agents, emulsion stabilizers, film formers, gel formers, odor-masking agents, resins, hydrocolloids, solvents, solubilizers, neutralizers, permeation promoters, pigments, colorants, stabilizers, disintegrants, dessicants, opacifiers, thickeners, waxes, plasticizers, flavors, sweeteners, excipients to reduce permeation etc. An arrangement concerning this is based on specialist knowledge as described for example in Fiedler, H. P. Lexikon der Hilfsstoffe für Pharmazie, Kosmetik und angrenzende Gebiete, 4th edition, Aulendorf: ECV-Editio-Cantor-Verlag, 1996.

### Microbiocidal composition

It was found surprisingly, that the modified polysaccharides A) according to the invention exhibit high affinity to various surfaces, in particular inorganic surfaces, such as metals, e.g. stainless steel, glass, ceramic, mineral materials, and polymer materials and are suitable for modifying the surface properties of these materials. Thus, the modified polysaccharides A) according to the invention are suitable to minimize the risk of microbial colonization of materials coated therewith or materials comprising those modified polysaccharides A) in incorporated form.

Infections with microorganisms represent a major problem e.g. in connection with medical instruments and devices, implant materials, wound protection films and dressing materials. Such infections are triggered by the adhesion of surface-active microorganisms and the resulting biofilm development primarily on hydrophobic surfaces. The adhesion of the bacteria to surfaces is based on the one hand on nonspecific interactions such as electrostatic interactions, Van-der-Waals forces and acid-base interactions and, on the other hand, on specific interactions such as receptor/ligand bonds. The surface of pathogenic bacteria is covered with adhesins, i.e. proteins which aid adhesion to surfaces. The nature of the surface of the materials such as roughness and surface tension are decisive criteria for the colonization of surfaces. For this reason, it is important, through a modification of the surfaces, to minimize the number of adhering bacteria and thus the plaque formation and the infection of adjacent tissue.

In a preferred embodiment, the modified polysaccharides A) according to the invention are used as antimicrobial coating. The coating according to the invention is in particular suitable for the coating of material surfaces in the medical-therapeutic application, for example for the coating of metallic implants, of wound protection films and dressing material or for the coating of medical instruments and devices, such as catheters. In the field of biotechnology, the coating according to the invention is suitable in particular for the construction of apparatuses (e.g. fermentors), for the coating of seals and for suppressing biofouling.

A further aspect of the invention is a microbiocidal composition, comprising at least modified polysaccharides according to the invention or obtainable by the process of the invention.

The content of the modified polysaccharide A) in the microbiocidal compositions of the invention can be varied over wide ranges. Preferably, the microbiocidal composition comprises the at least one modified polysaccharide A) in an amount of from 0.01 to 100 wt.%, more preferably 0.1 to 99.9 wt.%, in particular 0.5 to 95 wt.%, based on the total weight of the composition. Especially, the microbiocidal composition comprises the at least one modified polysaccharide A) in an amount of from 0.01 to 10 wt.%, more especially 0.05 to 5 wt.%, in particular 0.1 to 1 wt.%, based on the total weight of the composition.

In addition to at least one modified polysaccharide A) (= component A), the microbiocidal compositions of the invention may comprise at least one further microbiocidal compound different from the compounds of component (A) (= component B).

Suitable further microbiocidal compounds (B) are selected from
- alcohols, including halogenated alcohols,
- isothiazolones,
- activated halogen compounds,
- formaldehyde release compounds,
- phenolic compounds,
- aldehydes,
- acids and esters,
- biphenyls,
- urea derivatives,
- O-acetals, O-formals,
- N-acetals, N-formals,
- benzamidines,
- phthalimides,
- pyridine derivatives,
- quaternary ammonium and phosphonium compounds,
- amines,
- amphoteric compounds,
- dithiocarbamates,
- compounds containing active oxygen such as peroxide,
- inorganic salts such as metal oxides, metal chlorides, metal sulfates, etc.,
- organic metal salts, such as Zn-pyrithion, Ag-lactate, etc.,
- mixtures thereof.

Examples of alcohol compounds which may serve as the microbiocidally effective component (B) are 2-bromo-2-nitropropane-1,3-diol and 2-(hydroxymethyl)-2-nitro-1,3-propanediol. Examples of isothiazolone compounds are 5-chloro-2-methyl-2H-isothiazol-3-one (CIT), 2-methyl-2H-isothiazol-3-one (MIT), 1,2-benzisothiazol-3(2H)-one, 2-n-octyl-2H-isothiazol-3-one, 4,5-dichloro-2-octyl-2H-isothiazol-3-one and 2-butyl-benzo-[d]isothiazol-3-one and mixtures thereof with one another, including a mixture of CIT with MIT or mixtures of CIT or MIT with any of 1,2-benzoisothiazol-3(2H)-one, 2-octyl-2H-isothiazol-3-one, 4,5-dichloro-2-octyl-2H-isothiazol-3-one and 2-butyl-benzo[d]isothiazol-3-one. Examples of other compounds are dibromodicyanobutane, [beta]-bromo-[beta]-nitrostyrene, 7a-ethyldihydro-1 H,3H,5H-oxazolo[3,4-c]oxazole, tetrahydro-1,3,4,6-tetrakis(hydroxymethyl)-imidazo[4,5-d]-imidazole-2,5(1H,3H)-dione, 1,3-dimethyl-5,5-dimethylhydantoin, diazolidinyl ureas and imidazolidinyl ureas, N'-(3,4-dichlorophenyl)-N,N-dimethyl urea, 3,3'-methylene-bis(5-methyl-oxazolidine), 2-sodiumsulfidopyridine-N-oxide and its metal salts, dibromonitritopropionamide, tetrakishydroxymethylphosphonium salts, ortho-phenylphenol and salts of ortho-phenylphenol, 1-(3-chloroallyl)-3,5,7-triaza-1-azodiadamantane salts, (5-chloro-2,4-dichlorophenoxy)phenol, 3,4,4'-trichlorocarbanilide (triclocarban), o-benzo-p-chlorophenol, p-hydroxybenzoates, 2-(thiocyanomethylthio) benzothiazole, 3,5-dimethyl-1,3,5-thiadiazinane-2-thione, 2,4-dichlorobenzyl alcohol, chlorothalonil, methylenebis(thiocyanate), peracetic acid, 4,4-dimethyl-oxazolidine, phenoxyethanol, phenoxypropanol, 2,6-dimethyl-m-dioxan-4-ol-acetate, glutaraldehyde, glyoxal, ortho-phthalaldehyde, 4-(2-nitrobutyl)-morpholine, triazines such as 1,3,5-tris-(2-hydroxyethyl)-1,3,5-hexahydrotriazine, quaternary ammonium compounds such as benzalkoniumchloride, polyhexamethylenebiguanide salts, poly(oxyethylene(dimethylimino)ethylene(dimethylimino)-ethylene dichloride, chlorhexidine gluconate, chloroisocyanurates, halogenated hydantoins such as 1-bromo-3-chloro-5,5-dimethylhydantoin and polamines, such as polyvinylamine- and polyethylene imine derivatives. Further examples include IPBC, terbutryn, ziram, zineb, dichlofluanid, trichlofuanid, folpet, metal dihexa-2,4-dienoate, tebuconazole, 3-benzo(b)thien-2-yl-5,6-dihydro-1,4,2-oxathiazine, 4-oxide, pyrithiones, thiram, cybutryne, MBT, carbendazim, diuron, chlorotoluron, fluorometuron, thiabendazole, metazachlor, CuSCN, or dicopper oxide.

Preferred components (B) are 2-bromo-2-nitropropane-1,3-diol, 2-methyl-2H-isothiazol-3-one, 1,2-benzisothiazol-3(2H)-one, 2-n-octyl-2H-isothiazol-3-one, a mixture of 5-chloro-2-methyl-2H-isothiazol-3-one with 2-methyl-2H-isothiazol-3-one, dibromodicyanobutane, tetrahydro-1,3,4,6-tetrakis(hydroxymethyl)-imidazo[4,5-d]-imidazole-2,5(1H,3H)-dione, 3,3'-methylenebis(5-methyl-oxazolidine), 1,3-dimethyl-5,5-dimethylhydantoin, tetrakishydroxymethylphosphonium salts, ortho-phenylphenol and salts of ortho-phenylphenol, 1-(3-chloroallyl)-3,5,7-triaza-1-azodiadamantane salts, (5-chloro-2,4-dichlorophenoxy)phenol, 3,4,4'-trichlorocarbanilide (triclocarban), p-hydroxy-benzoates, 2-(thiocyanomethylthio) benzothiazole, 3,5-dimethyl-1,3,5-thiadiazinane-2-thione), iodo-2-propynylbutylcarbamate, 2-sodiumsulfidopyridine-N-oxide and its metal salts, 2,4-dichlorobenzyl alcohol, chlorothalonil, methylenebis(thiocyanate), phenoxyethanol, phenoxypropanol, triazines, such as 1,3,5-tris-(2-hydroxyethyl)-1,3,5-hexahydrotriazine, quaternary ammonium compounds, such as benzalkoniumchloride, polyhexamethylene biguanide salts, poly(oxyethylene(dimethyimino)ethylene (dimethylimino)ethylene dichloride, chlorhexidine gluconate, chloroisocyanurates and polyvinylamines, especially the polyamines disclosed in WO-A-97/32477.

If the microbiocidal composition according to the invention comprises components (A) and (B), the amounts of the components (A) and (B) in the composition are preferably 1 to 99 wt.% of (A) and 99 to 1 wt.% of (B), more preferably 10 to 90 wt.% of (A) and 90 to 10 wt.% of (B), especially 20 to 80 wt.% of (A) and 80 to 20 wt.% of (B). Preferably, the microbiocidal composition comprises the sum of compounds (A) and (B) in an amount of from 0.01 to 100 wt.%, more preferably 0.1 to 99.9 wt.%, in particular 0.5 to 95 wt.%, based on the total weight of the composition. Especially, the microbiocidal composition comprises the sum of compounds (A) and (B) in an amount of from 0.01 to 10 wt.%, more especially 0.05 to 5 wt.%, in particular 0.1 to 1 wt.%, based on the total weight of the composition.

The microbiocidal composition according to the invention, comprising a component (A), optionally (B) and optionally further components can be made up into the usual formulations and preparations that are suitable for the desired purpose. The microbiocidal composition according to the invention can be provided and/or applied as a solid or as a liquid. This encompasses compositions in form of aerosols. The microbiocidal composition according to the invention can be formulated e.g. as powder, granulate, pellets, pills, agglomerates, solutions, emulsions, suspensions, dispersions, pastes, in combination with carrier materials, etc.

The microbiocidal compositions according to the invention can be formulated free from solvent or with a suitable solvent. Generally, the imidazolium compounds used according to the invention are soluble in most protic solvents, swellable in most aprotic polar solvents and insoluble in most nonpolar solvents. Suitable solvents for the microbiocidal compositions according to the invention are selected from among water, alcohols, such as methanol, ethanol, n-propanol, isopropanol, n-butanol, tert-butanol, diols and polyols, such as ethanediol and propanediol, amino alcohols, such as ethanolamine, diethanolamine and triethanolamine, ethers, e.g. tetrahydrofuran, diethyl ether, methyl tert-butyl ether and diethylene glycol monomethyl ether, ketones, such as acetone and methyl ethyl ketone, esters, e.g. ethyl acetate, formamide, dimethylformamide (DMF), dimethylacetamide, dimethyl sulfoxide (DMSO), acetonitrile, aromatic solvents, e.g. benzene, toluene, ethylbenzene or xylenes, halogenated solvents, e.g. dichloromethane, chloroform, carbon tetrachloride, dichloroethane or chlorobenzene, aliphatic solvents, e.g. pentane, hexane, heptane, octane, ligroin, petroleum ether, cyclohexane and decalin, and mixtures thereof.

The solvent is preferably selected from among water, water-miscible organic solvents and mixtures thereof. The solvent is particularly preferably selected from among water, methanol, ethanol, n-propanol, isopropanol, n-butanol, tert-butanol and mixtures thereof.

A multitude of different active substances and additives can be formulated in the microbiocidal compositions according to the invention.

The modified polysaccharides A) employed according to the invention are in particular suitable to provide antimicrobial coating compositions, for example compositions for medical applications. The coating compositions show an outstanding antimicrobial activity. Thus, in a further aspect, the invention provides an antimicrobial coating composition, comprising an effective antimicrobial amount of at least one modified polysaccharide A), as defined above.

Preferably, the coating composition comprises at least one modified polysaccharide A) in an amount of from about 0.001 to about 15.0 weight percent, more preferably 0.01 to 10.0 weight percent, based on the total weight of the polymer composition or the coating composition.

A further aspect of the invention is a coating composition, comprising
A) at least one modified polysaccharide A) as defined above,
B) optionally at least one further microbiocidal compound different from the compounds of component (A),
C) optionally at least one polymer and/or at least one polymerizable compound, and
D) optionally at least one additive.

With regard to suitable and preferred compounds of the components A) and B), reference is made to the aforementioned description of suitable and preferred embodiments of those components.

The employed polymers may be in any form, for example fibers, films or molded parts. They may be for example woven or nonwoven polymer fabrics.

Suitable polymers C) may be selected from:
1. Polymers of monoolefins and diolefins, for example polypropylene, polyisobutylene, polybut-1-ene, poly-4-methylpent-1-ene, polyisoprene or polybutadiene, as well as polymers of cycloolefins, for instance of cyclopentene or norbornene, polyethylene (which optionally can be crosslinked), for example high density polyethylene (HDPE), low density polyethylene (LDPE), linear low density polyethylene (LLDPE), branched low density polyethylene (BLDPE) and medium density polyethylene (MDPE).
   Polyolefins, i.e. the polymers of monoolefins exemplified in the preceding paragraph, preferably polyethylene and polypropylene, can be prepared by different, and especially by the following, methods:
   a) radical polymerization (normally under high pressure and at elevated temperature).
   b) catalytic polymerization using a catalyst that normally contains one or more than one metal of groups IVb, Vb, Vlb or VIII of the Periodic Table. These metals usually have one or more than one ligand, typically oxides, halides, alcoholates, esters, ethers, amines, alkyls, alkenyls and/or aryls that may be either p- or s-coordinated. These metal complexes may be in the free form or fixed on substrates, typically on activated magnesium chloride, titanium(III) chloride, alumina or silicon oxide. These catalysts may be soluble or insoluble in the polymerization medium. The catalysts can be used by themselves in the polymerization or further activators may be used, typically metal alkyls, metal hydrides, metal alkyl halides, metal alkyl oxides or metal alkyloxanes, said metals being elements of groups la, IIa and/or IIIa of the Periodic Table. The activators may be modified conveniently with further ester, ether, amine or silyl ether groups. These catalyst systems are usually termed Phillips, Standard Oil Indiana, Ziegler (-Natta), TNZ (DuPont), metallocene or single site catalysts (SSC).
2. Mixtures of the polymers mentioned under 1), for example mixtures of polypropylene with polyisobutylene, polypropylene with polyethylene (for example PP/HDPE, PP/LDPE) and mixtures of different types of polyethylene (for example LDPE/HDPE).
3. Copolymers of monoolefins and diolefins with each other or with other vinyl monomers, for example ethylene/propylene copolymers, linear low density polyethylene (LLDPE) and mixtures thereof with low density polyethylene (LDPE), propylene/but-1-ene copolymers, propylene/isobutylene copolymers, ethylene/but-1-ene copolymers, ethylene/hexene copolymers, ethylene/methylpentene copolymers, ethylene/heptene copolymers, ethylene/octene copolymers, propylene/butadiene copolymers, isobutylene/isoprene copolymers, ethylene/alkyl acrylate copolymers, ethylene/alkyl methacrylate copolymers, ethylene/vinyl acetate copolymers and their copolymers with carbon monoxide or ethylene/acrylic acid copolymers and their salts (ionomers) as well as terpolymers of ethylene with propylene and a diene such as hexadiene, dicyclopentadiene or ethylidenenorbornene; and mixtures of such copolymers with one another and with polymers mentioned in 1) above, for example polypropylene/ethylene-propylene copolymers, LDPE/ethylene-vinyl acetate copolymers (EVA), LDPE/ethylene-acrylic acid copolymers (EAA), LLDPE/EVA, LLDPE/EAA and alternating or random polyalkylene/carbon monoxide copolymers and mixtures thereof with other polymers, for example polyamides.
4. Hydrocarbon resins (for example C5-C9) including hydrogenated modifications thereof (e.g. tackifiers) and mixtures of polyalkylenes and starch.
5. Polystyrene, poly(p-methylstyrene), poly(a-methylstyrene).
6. Copolymers of styrene or [alpha-methylstyrene with dienes or acrylic derivatives, for example styrene/butadiene, styrene/unsaturated ester, styrene/acrylonitrile, styrene/alkyl methacrylate, styrene/butadiene/alkyl acrylate, styrene/butadiene/alkyl methacrylate, styrene/maleic anhydride, styrene/acrylonitrile/methyl acrylate; mixtures of high impact strength of styrene copolymers and another polymer, for example a polyacrylate, a diene polymer or an ethylene/propylene/diene terpolymer; and block copolymers of styrene such as styrene/butadiene/styrene, styrene/isoprene/styrene, styrene/ethylene/butylene/styrene or styrene/ethylene/propylene/styrene.
7. Graft copolymers of styrene or [alpha-methylstyrene, for example styrene on polybutadiene, styrene on polybutadiene-styrene or polybutadiene-acrylonitrile copolymers; styrene and acrylonitrile (or methacrylonitrile) on polybutadiene; styrene, acrylonitrile and methyl methacrylate on polybutadiene; styrene and maleic anhydride on polybutadiene; styrene, acrylonitrile and maleic anhydride or maleimide on polybutadiene; styrene and maleimide on polybutadiene; styrene and alkyl acrylates or methacrylates on polybutadiene; styrene and acrylonitrile on ethylene/propylene/diene terpolymers; styrene and acrylonitrile on polyalkyl acrylates or polyalkyl methacrylates, styrene and acrylonitrile on acrylate/butadiene copolymers, as well as mixtures thereof with the copolymers listed under 6), for example the copolymer mixtures known as ABS, SAN, MBS, ASA or AES polymers.
8. Halogen-containing polymers such as polychloroprene, chlorinated rubbers, chlorinated or sulfochlorinated polyethylene, copolymers of ethylene and chlorinated ethylene, epichlorohydrin homo- and copolymers, especially polymers of halogen-containing vinyl compounds, for example polyvinyl chloride, polyvinylidene chloride, polyvinyl fluoride, polyvinylidene fluoride, as well as copolymers thereof such as vinyl chloride/vinylidene chloride, vinyl chloride/vinyl acetate or vinylidene chloride/vinyl acetate copolymers.
9. Polymers derived from α,β-unsaturated acids and derivatives thereof such as polyacrylates and polymethacrylates; polymethyl methacrylates, polyacrylamides and polyacrylonitriles, impact-modified with butyl acrylate.
10. Copolymers of the monomers mentioned under 9) with each other or with other unsaturated monomers, for example acrylonitrile/ butadiene copolymers, acrylonitrile/alkyl acrylate copolymers, acrylonitrile/alkoxyalkyl acrylate or acrylonitrile/vinyl halide copolymers or acrylonitrile/ alkyl methacrylate/butadiene terpolymers.
11. Polymers derived from unsaturated alcohols and amines or the acyl derivatives or acetals thereof, for example polyvinyl alcohol, polyvinyl acetate, polyvinyl stearate, polyvinyl benzoate, polyvinyl maleate, polyvinyl butyral, polyallyl phthalate or polyallyl melamine; as well as their copolymers with olefins mentioned in 1) above.
12. Homopolymers and copolymers of cyclic ethers such as polyalkylene glycols, polyethylene oxide, polypropylene oxide or copolymers thereof with bis-glycidyl ethers.
13. Polyacetals such as polyoxymethylene and those polyoxymethylenes which contain ethylene oxide as a comonomer; polyacetals modified with thermoplastic polyurethanes, acrylates or MBS.
14. Polyphenylene oxides and sulfides, and mixtures of polyphenylene oxides with styrene polymers or polyamides.
15. Polyurethanes derived from hydroxyl-terminated polyethers, polyesters or polybutadienes on the one hand and aliphatic or aromatic polyisocyanates on the other, as well as precursors thereof.
16. Polyamides and copolyamides derived from diamines and dicarboxylic acids and/or from aminocarboxylic acids or the corresponding lactams, for example polyamide 4, polyamide 6, polyamide 6/6, 6/10, 6/9, 6/12, 4/6, 12/12, polyamide 11, polyamide 12, aromatic polyamides starting from m-xylene diamine and adipic acid; polyamides prepared from hexamethylenediamine and isophthalic or/and terephthalic acid and with or without an elastomer as modifier, for example poly-2,4,4,-trimethylhexamethylene terephthalamide or poly-m-phenylene isophthalamide; and also block copolymers of the aforementioned polyamides with polyolefins, olefin copolymers, ionomers or chemically bonded or grafted elastomers; or with polyethers, e.g. with polyethylene glycol, polypropylene glycol or polytetramethylene glycol; as well as polyamides or copolyamides modified with EPDM or ABS; and polyamides condensed during processing (RIM polyamide systems).
17. Polyureas, polyimides, polyamide-imides and polybenzimidazoles.
18. Polyesters derived from dicarboxylic acids and diols and/or from hydroxycarboxylic acids or the corresponding lactones, for example polyethylene terephthalate, polytrimethylene terephthalate, polybutylene terephthalate, poly-1,4-dimethylolcyclohexane terephthalate and polyhydroxybenzoates, as well as block copolyether esters derived from hydroxyl-terminated polyethers; and also polyesters modified with polycarbonates or MBS. Polyesters and polyester copolymers as defined in U.S. Pat. No. 5,807,932 (column 2, line 53), incorporated herein by reference.
19. Polycarbonates and polyester carbonates.
20. Polysulfones, polyether sulfones and polyether ketones.
21. Crosslinked polymers derived from aldehydes on the one hand and phenols, ureas and melamines on the other hand, such as phenol/formaldehyde resins, urea/formaldehyde resins and melamine/formaldehyde resins.
22. Drying and non-drying alkyd resins.
23. Unsaturated polyester resins derived from copolyesters of saturated and unsaturated dicarboxylic acids with polyhydric alcohols and vinyl compounds as crosslinking agents, and also halogen-containing modifications thereof of low flammability.
24. Crosslinkable acrylic resins derived from substituted acrylates, for example epoxy acrylates, urethane acrylates or polyester acrylates.
25. Alkyd resins, polyester resins and acrylate resins crosslinked with melamine resins, urea resins, polyisocyanates or epoxy resins.
26. Crosslinked epoxy resins derived from polyepoxides, for example from bis glycidyl ethers or from cycloaliphatic diepoxides.
27. Natural polymers such as cellulose, rubber, gelatin and chemically modified homologous derivatives thereof, for example cellulose acetates, cellulose propionates and cellulose butyrates, or the cellulose ethers, such as methyl cellulose; as well as rosins and their derivatives.
28. Blends of the aforementioned polymers (polyblends), for example PP/EPDM, Polyamide/-EPDM or ABS, PVC/EVA, PVC/ABS, PVC/MBS, PC/ABS, PBTP/ABS, PC/ASA, PC/PBT, PVC/CPE, PVC/acrylates, POM/thermoplastic PUR, PC/thermoplastic PUR, POM/acrylate, POM/MBS, PPO/HIPS, PPO/PA 6.6 and copolymers, PA/HDPE, PA/PP, PA/PPO.
29. Naturally occurring and synthetic organic materials which are pure monomeric compounds or mixtures of such compounds, for example mineral oils, animal and vegetable fats, oil and waxes, or oils, fats and waxes based on synthetic esters (e.g. phthalates, adipates, phosphates or trimellitates) and also mixtures of synthetic esters with mineral oils in any weight ratios, typically those used as spinning compositions, as well as aqueous emulsions of such materials.
30. Aqueous emulsions of natural or synthetic rubber, e.g. natural latex or latices of carboxylated styrene/butadiene copolymers.
31. Polysiloxanes such as the soft, hydrophilic polysiloxanes described, for example, in U.S. Pat. No. 4,259,467; and the hard polyorganosiloxanes described, for example, in U.S. Pat. No. 4,355,147.
32. Silicone elastomers, for example liquid silicone rubbers (LSR). Liquid Silicone Rubbers are available from Dow Chemical and are described for example in U.S. Pat. Nos. 6,569,536; 6,420,038; 6,297,291; 6,218,466; 6,130,272; 5,994,461; 5,989,719; 5,973,030; 5,908,888; 5,880,199; 5,877,256; 5,859,094; 5,789,084 and 5,661,210. The disclosures of these U.S. patents are incorporated by reference.
33. Polyketimines in combination with unsaturated acrylic polyacetoacetate resins or with unsaturated acrylic resins. The unsaturated acrylic resins include the urethane acrylates, polyether acrylates, vinyl or acryl copolymers with pendant unsaturated groups and the acrylated melamines. The polyketimines are prepared from polyamines and ketones in the presence of an acid catalyst.
34. Radiation curable compositions containing ethylenically unsaturated monomers or oligomers and a polyunsaturated aliphatic oligomer.
35. Epoxymelamine resins such as light-stable epoxy resins crosslinked by an epoxy functional coetherified high solids melamine resin such as LSE-4103 (Monsanto).

Included also are thermoplastic olefin (TPO), thermoplastic elastomers, polyetherimide, polymethylpentene, polyphenylene ether, polyphenylene sulfide, polysulfone or polytetrafluoroethylene (PTFE).

Some polymers of specific technical interest include:
Polysulfone (PSF)
Polyethersulfone (PES)
Polyphenylsulfone (PPS)
Polyvinylidene Fluoride (PVDF)
Polypropylene (PP)
Polyethylene (PE)
Cellulose, Cellulose acetates (CA), Cellulose nitrate
Polyamide (PA)
Polyacrylonitrile (PAN)
Polytetrafluoroethylene (PTFE)
Polycarbonate (PC)
Polymethylmethacrylate (PMMA)

In particular, the present polymers are those that are typically employed in medical applications, for example polyurethanes, polycarbonate, liquid silicone rubbers, polyethylene, polypropylene, polyethylene/polypropylene copolymers or polymer composites.

Polymer composites are for instance natural products composites, for example a natural product mixed with a thermoplastic polymer such as a polyolefin. Such composites are disclosed in published U.S. app. No. 20040235983, the disclosure of which is hereby incorporated by reference. Natural products are for instance wood flour, flax, hemp, jute, kenaf or rice husk. The thermoplastic polymer is for instance polyethylene or polypropylene.

A preferred polymer composition or coating composition according to the invention additionally contains, for example, one or more components D) selected from antioxidants, light stabilizers (such as UV absorbers and/or sterically hindered amines, phosphites, phosphonites), metal deactivators, nucleating agents, fillers, plasticisers, pigments, flameproofing agents, antistatic agents, lubricants, emulsifiers, rheology additives, catalysts, flow-control agents, optical brighteners, blowing agents and combinations thereof.

The employed components D), in particular the antioxidants, light stabilizers, and metal deactivators, preferably have a high migration fastness and temperature resistance.

Suitable antioxidants D) are selected from the following classes:
1. Alkylated monophenols, for example 2,6-di-tert-butyl-4-methylphenol, 2-tert-butyl-4,6-di-methylphenol, 2,6-di-tert-butyl-4-ethylphenol, etc.;
2. Alkylthiomethylphenols, for example 2,4-dioctylthiomethyl-6-tert-butylphenol, 2,4-dioctylthiomethyl-6-methylphenol, 2,4-dioctylthiomethyl-6-ethylphenol, 2,6-di-dodecylthiomethyl-4-nonylphenol, etc.;
3. Hydroquinones and alkylated hydroquinones, for example 2,6-di-tert-butyl-4-methoxyphenol, 2,5-di-tert-butylhydroquinone, 2,5-di-tert-amylhydroquinone, 2,6-diphenyl-4-octadecyloxyphenol, 2,6-di-tert-butylhydroquinone, 2,5-di-tert-butyl-4-hydroxyanisole, 3,5-di-tert-butyl-4-hydroxyanisole, 3,5-di-tert-butyl-4-hydroxyphenyl stearate, bis(3,5-di-tert-butyl-4-hydroxyphenyl)adipate, etc.;
4. Tocopherols;
5. Hydroxylated thiodiphenyl ethers, for example 2,2'-thiobis(6-tert-butyl-4-methylphenol), 2,2'-thiobis(4-octylphenol), 4,4'-thiobis(6-tert-butyl-3-methylphenol), 4,4'-thiobis(6-tert-butyl-2-methylphenol), 4,4'-thiobis(3,6-di-sec-amylphenol), 4,4'-bis(2,6-dimethyl-4-hydroxyphenyl)-disulfide, etc.;
6. Alkylidenebisphenols, for example 2,2'-methylenebis(6-tert-butyl-4-methyl-phenol), 2,2'methylenebis(6-tert-butyl-4-ethylphenol), 2,2'-methylenebis[4-methyl-6-(α-methylcyclohexyl)-phenol, 1,1-bis-(3,5-dimethyl-2-hydroxyphenyl)butane, 2,2-bis(3,5-di-tert-butyl-4-hydroxyphenyl)propane, 2,2-bis(5-tert-butyl-4-hydroxy-2-methylphenyl)-4-n-dodecylmercaptobutane, 1,1,5,5-tetra-(5-tert-butyl-4-hydroxy-2-methylphenyl)pentane, etc.;
7. O-, N- and S-benzyl compounds, for example 3,5,3',5'-tetra-tert-butyl-4,4'-dihydroxydibenzyl ether, octadecyl-4-hydroxy-3,5-dimethylbenzylmercapto-acetate, tridecyl-4-hydroxy-3,5-di-tert-butylbenzylmercaptoacetate, tris(3,5-di-tert-butyl-4-hydroxybenzyl)amine, bis(4-tert-butyl-3-hydroxy-2,6-dimethylbenzyl)-dithioterephthalate, bis(3,5-di-tert-butyl-4-hydroxybenzyl)sulfide, isooctyl-3,5-di-tert-butyl-4-hydroxybenzylmercaptoacetate, etc.;
8. Hydroxybenzylated malonates, for example dioctadecyl-2,2-bis(3,5-di-tert-butyl-2-hydroxybenzyl)malonate, di-octadecyl-2-(3-tert-butyl-4-hydroxy-5-methylbenzyl)-malonate, didodecylmercaptoethyl-2,2-bis(3,5-di-tert-butyl-4-hydroxybenzyl)-malonate, bis[4-(1,1,3,3-tetramethylbutyl)phenyl-2,2-bis(3,5-di-tert-butyl-4-hydroxybenzyl)malonate, etc.;
9. Aromatic hydroxybenzyl compounds, for example 1,3,5-tris(3,5-di-tert-butyl-4-hydroxybenzyl)-2,4,6-trimethylbenzene, 1,4-bis(3,5-di-tert-butyl-4-hydroxy-benzyl)-2,3,5,6-tetramethylbenzene, 2,4,6-tris(3,5-di-tert-butyl-4-hydroxybenzyl)-phenol, etc.;
10. Triazine compounds, for example 2,4-bis(octylmercapto)-6-(3,5-di-tert-butyl-4-hydroxy-anilino)-1,3,5-triazine, 2-octylmercapto-4,6-bis(3,5-di-tert-butyl-4-hydroxyanilino)-1,3,5-triazine, 2-octylmercapto-4,6-bis(3,5-di-tert-butyl-4-hydroxyphenoxy)-1,3,5-triazine, 2,4,6-tris-(3,5-di-tert-butyl-4-hydroxyphenoxy)-1,2,3-triazine, 1,3,5-tris(3,5-di-tert-butyl-4-hydroxybenzyl)isocyanurate, 1,3,5-tris(4-tert-butyl-3-hydroxy-2,6-dimethylbenzyl)isocyanurate, 2,4,6-tris-(3,5-di-tert-butyl-4-hydroxyphenylethyl)-1,3,5-triazine, 1,3,5-tris(3,5-di-tert-butyl-4-hyphenylpropionyl)-hexahydro-1,3,5-triazine, 1,3,5-tris(3,5-dicyclohexyl-4-hydroxybenzyl)isocyanurate, etc.;
11. Benzylphosphonates, for example dimethyl-2,5-di-tert-butyl-4-hydroxybenzylphosphonate, diethyl-3,5-di-tert-butyl-4-hydroxybenzylphosphonate, dioctadecyl3,5-di-tert-butyl-4-hydroxybenzylphosphonate, dioctadecyl-5-tert-butyl-4-hydroxy-3-methylbenzylphosphonate, the calcium salt of the monoethyl ester of 3,5-di-tert-butyl-4-hydroxybenzylphosphonic acid, etc.;
12. Acylaminophenols, for example 4-hydroxylauranilide, 4-hydroxystearanilide, octyl N-(3,5-di-tert-butyl-4-hydroxyphenyl)carbamate, etc.;
13. Esters of β-(3,5-di-tert-butyl-4-hydroxyphenyl)propionic acid with mono- or polyhydric alcohols;
14. Esters of β-(5-tert-butyl-4-hydroxy-3-methylphenyl)propionic acid with mono- or polyhydric alcohols;
15. Esters of β-(3,5-dicyclohexyl-4-hydroxyphenyl)propionic acid with mono- or polyhydric alcohols;
16. Esters of 3,5-di-tert-butyl-4-hydroxyphenyl acetic acid with mono- or polyhydric alcohols;
   Suitable mono- or polyhydric alcohols for compounds 13.) to 16.) are methanol, ethanol, octanol, octadecanol, 1,6-hexanediol, 1,9-nonanediol, ethylene glycol, 1,2-propanediol, neopentyl glycol, thiodiethylene glycol, diethylene glycol, triethylene glycol, pentaerythritol, tris(hydroxyethyl)isocyanurate, N,N'-bis-(hydroxyethyl)oxamide, 3-thiaundecanol, 3-thiapentadecanol, trimethylhexanediol, trimethylolpropane, 4-hydroxymethyl-1-phospha-2,6,7-trioxabicyclo[2.2.2octane.
17. Amides of β-(3,5-di-tert-butyl-4-hydroxyphenyl)propionic acid e.g. N,N'-bis(3,5-di-tert-butyl-4-hydroxyphenylpropionyl)hexamethylenediamide, N,N'-bis(3,5-di-tert-butyl-4-hydroxy-phenylpropionyl)trimethylenediamide, N,N'-bis(3,5-di-tert-butyl-4-hydroxyphenylpropionyl)hydrazide, N,N'-bis[2-(3-(3,5-di-tert-butyl-4-hydroxy-phenylpropionyloxy)ethyloxamide (Naugard(R)XL-1, supplied by Uniroyal).
18. Ascorbic Acid (Vitamin C)
19. Aminic antioxidants, for example N,N'-di-isopropyl-p-phenylenediamine, N,N'-di-sec-butyl-p-phenylenediamine, N,N'-bis(1,4-dimethylpentyl)-p-phenylenediamine, N,N'-bis(1-ethyl-3-methylpentyl)-p-phenylenediamine, N,N'-bis(1-methylheptyl)-p-phenylenediamine, N,N'-dicyclohexyl-p-phenylenediamine, N,N'-diphenyl-p-phenylenediamine, N,N'-bis(2-naphthyl)-p-phenylenediamine, N-isopropyl-N'-phenyl-p-phenylenediamine, N-(1,3-dimethylbutyl)-N'-phenyl-p-phenylene-diamine, N-(1-methylheptyl)-N'-phenyl-p-phenylenediamine, N-cyclohexyl-N'-phenyl-p-phenylenediamine, 4-(p-toluenesulfamoyl)diphenylamine, N,N'-dimethyl-N,N'-di-sec-butyl-p-phenylenediamine, diphenylamine, N-allyldiphenylamine, 4-isopropoxydiphenyl-amine, N-phenyl-1-naphthylamine, N-(4-tert-octylphenyl)-1-naphthylamine, N-phenyl-2-naphthylamine, octylated diphenylamine, for example p,p'-di-tert-octyldiphenylamine, 4-n-butyl-aminophenol, 4-butyrylaminophenol, 4-nonanoylaminophenol, 4-dodecanoylaminophenol, 4-octadecanoylaminophenol, bis(4-methoxy-phenyl)amine, 2,6-di-tert-butyl-4-dimethylaminomethylphenol, 2,4'-diamino-diphenylmethane, 4,4'-diaminodiphenylmethane, N,N,N',N'-tetramethyl-4,4'-diaminodiphenylmethane, 1,2-bis[(2-methylphenyl)aminoethane, 1,2-bis(phenyl-amino)propane, (o-tolyl)biguanide, bis[4-(1',3'-dimethylbutyl)phenylamine, tert-octylated N-phenyl-1-naphthylamine, a mixture of mono- and dialkylated tert-butyl/tert-octyldiphenylamines, a mixture of mono- and dialkylated nonyldiphenylamines, a mixture of mono- and dialkylated dodecyldiphenylamines, a mixture of mono- and
   dialkylatedisopropyl/isohexyldiphenylamines, a mixture of mono- and dialkylated tert-butyldiphenylamines, 2,3-dihydro-3,3-dimethyl-4H-1,4-benzothiazine, phenothiazine, a mixture of mono- and dialkylated tert-butyl/tert-octylphenothiazines, a mixture of mono- and dialkylated tert-octyl-phenothiazines, N-allylphenothiazine, N,N,N',N'-tetraphenyl-1,4-diaminobut-2-ene.

Suitable light stabilizers (UV absorbers) and metal deactivators are selected, for example, from groups a) to s):
a) 4,4-diarylbutadienes,
b) cinnamic esters,
c) benzotriazoles,
d) hydroxybenzophenones,
e) diphenylcyanoacrylates,
f) oxamides,
g) 2-phenyl-1,3,5-triazines,
h) antioxidants,
i) nickel compounds,
j) sterically hindered amines,
k) metal deactivators,
l) phosphites and phosphonites,
m) hydroxylamines,
n) nitrones,
o) amine oxides,
p) benzofuranones and indolinones,
q) thiosynergists,
r) peroxide scavengers, and
s) basic costabilizers.

The modified polysaccharides A), optional antimicrobial compounds B) and optional additives D) may be added to at least one polymer and/or at least one polymerizable compound C) individually or mixed with one another. If desired, the individual components can be mixed with one another before incorporation into the polymer or polymerizable composition for example by dry blending.

The incorporation of the biocides of the invention can be carried out by known methods, such as dry blending in the form of a powder or wet mixing in the form of solutions, dispersions or suspensions for example in an inert solvent, water or oil. The biocides of the invention may be incorporated, for example, before or after molding or also by applying the dissolved or dispersed biocide (or an additive mixture containing the biocide) to the polymer material or the polymerizable composition, with or without subsequent evaporation of the solvent or the suspension/dispersion agent. They may be added directly into the processing apparatus (e.g. extruders, internal mixers, etc.), e.g. as a dry mixture or powder or as solution or dispersion or suspension.

The incorporation can be carried out e.g. in any heatable container equipped with a stirrer, e.g. in a closed apparatus such as a kneader, mixer or stirred vessel. The incorporation is preferably carried out in an extruder or in a kneader. The processing may take place in an inert atmosphere or in the presence of oxygen.

The addition of biocide (or an additive mixture containing the biocide) to the polymer substrate can be carried out in all customary mixing machines in which the polymer is melted and mixed with the additives. Suitable machines are known to those skilled in the art. They are predominantly mixers, kneaders and extruders.

Processing includes extrusion, co-kneading, pultrusion, compression molding, sheet extrusion, thermoforming, injection molding or rotational molding. The process is preferably carried out in an extruder by introducing the additives during processing.

Particularly preferred processing machines are single-screw extruders, contrarotating and corotating twin-screw extruders, rotomolding devices, planetary-gear extruders, ring extruders or cokneaders. It is also possible to use processing machines provided with at least one gas removal compartment to which a vacuum can be applied.

Suitable extruders and kneaders are described, for example, in Handbuch der Kunststoffextrusion, Vol. 1 Grundlagen, Editors F. Hensen, W. Knappe, H. Potente, 1989, pp. 3-7, ISBN:3-446-14339-4 (Vol. 2 Extrusionsanlagen 1986, ISBN 3-446-14329-7).

The biocide (or an additive mixture containing the biocide) can also be added to the polymer in the form of a masterbatch ("concentrate") which contains the components in a concentration of, for example, about 1 % to about 40% and preferably about 2% to about 20% by weight incorporated in a polymer. The polymer must not necessarily be identical to the polymer where the additives are added finally. In such operations, the polymer can be used in the form of powder, granules, solutions, suspensions or in the form of latices. Incorporation can take place prior to or during the shaping operation, or by applying the dispersed compound to the polymer, with or without subsequent evaporation of the solvent. A further possibility for incorporating the biocide of the invention (or an additive mixture containing the biocide) into polymer substrates is to add them before, during or directly after the polymerization of the corresponding monomers or prior to crosslinking. In this context the additives of the invention can be added as it is or else in encapsulated form (for example in waxes, oils or polymers).

The polymers containing the biocide (or an additive mixture containing the biocide) described herein can be used for the production of moldings, rotomolded articles, injection molded articles, blow molded articles, profiles, films, woven and nonwoven fabrics, and the like.

When the polymer composition or coating composition according to the invention is used in the medical sector, it is for example a catheter, hose, tube, valve, articles for urology, bone cement, fabric, toothbrushes, silicone plastics, films, textiles, diapers and the like.

The coating composition according to the invention usually contains a film forming binder.

The binder can in principle be any binder which is customary in industry, for example those described in Ullmann's Encyclopedia of Industrial Chemistry, 5th Edition, Vol. A18, pp. 368-426, VCH, Weinheim 1991. In general, it is a film forming binder based on a thermoplastic or thermosetting resin, predominantly on a thermosetting resin. Examples thereof are alkyd, acrylic, acrylic alkyd, polyester, phenolic, melamine, epoxy and polyurethane resins and mixtures thereof. The binder can be a cold-curable or hot-curable binder; the addition of a curing catalyst may be advantageous. Suitable catalysts which accelerate curing of the binder are described, for example, in Ullmann's Encyclopedia of Industrial Chemistry, Vol. A18, p. 469, VCH Verlagsgesellschaft, Weinheim 1991.

The coating compositions according to the invention are for example employed as a top coat for plastics or metal or as a wood coating.

Examples of coatings compositions containing specific binders are:
1. paints based on cold- or hot-crosslinkable alkyd, acrylate, polyester, epoxy or melamine resins or mixtures of such resins, if desired with addition of a curing catalyst;
2. two-component polyurethane paints based on hydroxyl-containing acrylate, polyester or polyether resins and aliphatic or aromatic isocyanates, isocyanurates or polyisocyanates;
3. one-component polyurethane paints based on blocked isocyanates, isocyanurates or polyisocyanates which are deblocked during baking, if desired with addition of a melamine resin;
4. one-component polyurethane paints based on a trisalkoxycarbonyltriazine crosslinker and a hydroxyl group containing resin such as acrylate, polyester or polyether resins;
5. one-component polyurethane paints based on aliphatic or aromatic urethaneacrylates or polyurethaneacrylates having free amino groups within the urethane structure and melamine resins or polyether resins, if necessary with curing catalyst;
6. two-component paints based on (poly)ketimines and aliphatic or aromatic isocyanates, isocyanurates or polyisocyanates;
7. two-component paints based on (poly)ketimines and an unsaturated acrylate resin or a polyacetoacetate resin or a methacrylamidoglycolate methyl ester;
8. two-component paints based on carboxyl- or amino-containing polyacrylates and polyepoxides;
9. two-component paints based on acrylate resins containing anhydride groups and on a polyhydroxy or polyamino component;
10. two-component paints based on acrylate-containing anhydrides and polyepoxides;
11. two-component paints based on (poly)oxazolines and acrylate resins containing anhydride groups, or unsaturated acrylate resins, or aliphatic or aromatic isocyanates, isocyanurates or polyisocyanates;
12. two-component paints based on unsaturated polyacrylates and polymalonates;
13. thermoplastic polyacrylate paints based on thermoplastic acrylate resins or externally crosslinking acrylate resins in combination with etherified melamine resins;
14. paint systems based on siloxane-modified or fluorine-modified acrylate resins.

The coating compositions may also comprise further components, examples being solvents, pigments, dyes, plasticizers, stabilizers, thixotropic agents, drying catalysts and/or levelling agents. Examples of possible components are those described in Ullmann's Encyclopedia of Industrial Chemistry, 5th Edition, Vol. A18, pp. 429-471, VCH, Weinheim 1991.

Possible drying catalysts or curing catalysts are, for example, organometallic compounds, amines, amino-containing resins and/or phosphines. Examples of organometallic compounds are metal carboxylates, especially those of the metals Pb, Mn, Co, Zn, Zr or Cu, or metal chelates, especially those of the metals Al, Ti or Zr, or organometallic compounds, such as organotin compounds, for example.

Examples of metal carboxylates are the stearates of Pb, Mn or Zn, the octoates of Co, Zn or Cu, the naphthenates of Mn and Co or the corresponding linoleates, resinates or tallates.

Examples of metal chelates are the aluminium, titanium or zirconium chelates of acetylacetone, ethyl acetylacetate, salicylaldehyde, salicylaldoxime, o-hydroxyacetophenone or ethyl trifluoroacetylacetate, and the alkoxides of these metals.

Examples of organotin compounds are dibutyltin oxide, dibutyltin dilaurate or dibutyltin dioctoate.

Examples of amines are, in particular, tertiary amines, for example tributylamine, triethanolamine, N-methyldiethanolamine, N-dimethylethanolamine, N-ethylmorpholine, N-methylmorpholine or diazabicyclooctane (triethylenediamine) and salts thereof. Further examples are quaternary ammonium salts, for example trimethylbenzyl-ammonium chloride.

Amino-containing resins are simultaneously binder and curing catalyst. Examples thereof are amino-containing acrylate copolymers.

The curing catalyst used can also be a phosphine, for example triphenylphosphine.

The coating compositions can also be radiation-curable coating compositions. In this case, the binder essentially comprises monomeric or oligomeric compounds containing ethylenically unsaturated bonds, which after application are cured by actinic radiation, i.e. converted into a crosslinked, high molecular weight form. Where the system is UV-curing, it generally contains a photoinitiator as well. Corresponding systems are described in the above mentioned publication Ullmann's Encyclopedia of Industrial Chemistry, 5th Edition, Vol. A18, pages 451-453.

The coating compositions according to the invention can be applied to any desired substrates, for example to metal, wood, plastic, ceramic materials or plastic wood composites.

The coating compositions according to the invention are also suitable for protecting a wood surface. In this case, the coating compositions can be applied in form of a varnish, paint, etc. or by impregnation. A further aspect of the invention is a method for providing antimicrobial activity to a wood surface which method comprises applying a present coatings composition, especially a varnish, paint, stain or impregnation on wood. The coating composition may be applied by impregnation or as base coat (primer) or top coat.

If the coating composition is employed for protecting a wood surface, preferably a solvent is used, selected e.g. from the group consisting of aliphatic hydrocarbons, cycloaliphatic hydrocarbons, aromatic hydrocarbons, alcohols, ethers, esters, ketones, glycols, glycol ethers, glycol esters, polyglycols or mixtures thereof. Preferably, in this case the binder is selected from the group consisting of alkyd resins, modified alkyd resins, autocrosslinking or non-autocrosslinking acrylic resins, polyester resins, drying oils, phenolic resins, nitrocellulose or mixtures thereof.

Other additives like fungicides or insecticides are possible. Suitable components are known to the skilled artisan.

Any coating composition suitable for coating wood may be used as a top coat. It will normally contain a binder, dissolved or dispersed in an organic solvent or in water or a mixture of water and solvent. The binder may typically be a surface coating resin which dries in the air or hardens at room temperature. Exemplary of such binders are nitrocellulose, polyvinyl acetate, polyvinyl chloride, unsaturated polyester resins, polyacrylates, polyurethanes, epoxy resins, phenolic resins, and especially alkyd resins. The binder may also be a mixture of different surface coating resins. Provided the binders are curable binders, they are normally used together with a hardener and/or accelerator.

The top coat may also be a radiation-curable, solvent-free formulation of photopolymerizable compounds. Illustrative examples are mixtures of acrylates or methacrylates, unsaturated polyester/styrene mixtures or mixtures of other ethylenically unsaturated monomers or oligomers.

The top coat may contain a soluble dye and/or a pigment and/or a filler. The pigment may be an organic, inorganic or metallic pigment. The pigments may be opaque or transparent, such as for example transparent iron oxides. The filler may be typically kaolin, calcium carbonate or aluminium silicate. Preferably, the top coat is a clear varnish, i.e. it contains no undissolved components.

The present invention is particularly useful for the following applications: in home applications, such as furniture, wood floors, chipboards or timber work; outdoor applications, such as fences, construction parts, wooden fronts, window frames and the like.

The present coatings compositions may be applied to the substrates by the customary methods, for example by brushing, spraying, pouring, dipping or electrophoresis; see also Ullmann's Encyclopedia of Industrial Chemistry, 5th Edition, Vol. A18, pp. 491-500.

Depending on the binder system, the coatings may be cured at room temperature or by heating. The coatings may for example be cured at 50 to 150°C, and, e.g. in the case of powder coatings or coil coatings, even at higher temperatures.

The coating compositions can comprise an organic solvent or solvent mixture in which the binder is soluble. The coating compositions can otherwise be an aqueous solution or dispersion. The vehicle can also be a mixture of organic solvent and water. The coating composition may be a high-solids paint or can be solvent-free (e.g. a powder coating material). Powder coatings are, for example, those described in Ullmann's Encyclopedia of Industrial Chemistry, 5th Ed., A18, pages 438-444. The powder coating material may also have the form of a powder-slurry (dispersion of the powder preferably in water).

The pigments can be inorganic, organic or metallic pigments. The present coatings compositions may contain no pigments and may be used as a clearcoat.

The invention is illustrated in detail with reference to the following non-restrictive examples.

### EXAMPLES

### Example 1:

### Reaction of 6-deoxychlorocellulose with n-butylamine

### Reactants:

- 71.5 g (0.397 mol): 6-deoxychlorocellulose
- 1000 ml: dimethylformamide
- 300 ml: desalinated water
- 431.5 g (5.899 mol): n-butylamine

6-deoxychlorocellulose was prepared according to example 1 of US 2011/0175023 A1.

In a heated 2 L four-necked flask equipped with thermometer, cooler, dropping funnel, stirrer and nitrogen inlet the 6-deoxychlorocellulose was dissolved in dimethylformamide at 60°C. After stirring the reaction mixture for 1 hour 300 ml water were added and the mixture stirred for further 15 minutes while cooling down to ambient temperature. Afterwards the n-butylamine was slowly added under stirring. The reaction mixture was heated to a temperature of 100°C for 16 hours and then allowed to cool to ambient temperature. The reaction liquid became turbid and after addition of 2000 ml water a solid precipitated. The solid was isolated by filtration under reduced pressure, washed with water and dried.

### Yield: 55 g

### ¹³C CP MAS NMR:

| δ | signal |
|---|---|
| 103 ppm | cellulose C1 |
| 90 - 70ppm | cellulose C2-C5 |
| 59 ppm | C6_{Cellulose}-NH-***CH₂***-CH₂-CH₂-CH₃ |
| 50 ppm | cellulose C6 |
| 36 ppm | DMF |
| 31 ppm | C6_{Cellulose-}NH-CH₂-***CH₂***-CH₂-CH₃ + DMF |
| 20 ppm | C6_{Cellulose}-NH-CH₂-CH₂-***CH₂***-CH₃ |
| 14 ppm | C6_{Cellulose}-NH-CH₂-CH₂-CH₂-***CH₃*** |

### Example 2:

### reaction of 6-deoxychlorocellulose with ethyleneglycol

### Reactants:

| | |
|---|---|
| 5.0 g (28 mmol) | 6-deoxychlorocellulose |
| 4.6 g (82 mmol) | KOH |
| 50.0 g (820 mmol) | ethyleneglycol |

A heated 250 mL four-necked flask equipped with thermometer, cooler, dropping funnel, stirrer and nitrogen inlet was filled with the 6-deoxychlorocellulose, ethyleneglycol and KOH. Afterwards the mixture was heated to 80°C and kept at this temperature for 120 hours. After cooling to ambient temperature 150 mL methanol and 100 mL aceton were added. A fine solid precipitated that was isolated by filtration with a glass filter P4 under reduced pressure, washed twice with 25 ml methanol / 5% H₂O and dried at a temperature of 50°C under reduced pressure.

### Yield: 3.0 g

The structure was confirmed by ¹³C CP MAS NMR. The product showed no peak at 45 ppm corresponding to the cellulose C6 atom of the chlorinated educt.

### Example 3:

### Reaction of 6-deoxychlorocellulose with ethylenediamine

### Reactants:

| | |
|---|---|
| 10.0 g (55 mmol) | 6-deoxychlorocellulose |
| 100.0 g (1666 mmol) | ethylenediamine |

A heated 250 mL four-necked flask equipped with thermometer, cooler, dropping funnel, stirrer and nitrogen inlet was filled with the 6-deoxychlorocellulose and ethylenediamine. Afterwards the mixture was heated to 100°C and kept at this temperature for 1 hour. Then the reaction mixture was stirred at 80°C for further 96 hours. After cooling to ambient temperature the reaction mixture was added to 400 mL THF under stirring. The resulting solid was isolated by filtration under reduced pressure, washed and dried at a temperature of 80°C in high vacuum. Afterwards the resulting product was stirred with 400 ml ethanol at 25°C, isolated by filtration under reduced pressure, washed twice with 20 mL ethanol and dried again at a temperature of 80°C in high vacuum.

### Yield: 3.0 g

| elemental analysis | Cl % | C % | O % | N % | H % |
|---|---|---|---|---|---|
| product | 0 | 46,4 | 32,9 | 13,5 | 8,1 |
| theory | 0 | 47,1 | 31,4 | 13,7 | 7,8 |

The structure was confirmed by ¹³C CP MAS NMR. The product showed no peak at 45 ppm corresponding to the cellulose C6 atom of the chlorinated educt.

### ¹³C CP MAS NMR:

| δ | signal |
|---|---|
| 103 ppm | cellulose C1 |
| 90 - 65 ppm | cellulose C2-C5 |
| 50 ppm | cellulose C6 + C6_{Cellulose}-NH-***CH₂***-CH₂NH₂ |
| 40 ppm | C6_{Cellulose}-NH-CH₂-***CH₂***NH₂ |

### Example 4:

### Reaction of 6-deoxychlorocellulose with ethylenediamine

### Reactants:

| | |
|---|---|
| 561.0 g (3.06 mol) | 6-deoxychlorocellulose |
| 1850.0 g (303 mmol) | ethylenediamine |
| 1400.0 g | dimethylsulfoxide (DMSO) |

A heated 10 L reactor equipped with thermometer, dropping funnel, metal stirrer and distillation device was filled with the ethanolamine and DMSO and heated to 100°C. Afterwards 6-deoxychlorocellulose was added and the resulting mixture was kept at 100°C for 68 hours. Then the reaction mixture was stirred at 80°C for further 96 hours. After cooling to 75°C the reaction mixture was added to 2000 mL ethanol under stirring. Then the mixture was cooled to 30°C, divided in three portions and each portion added to 5 L aceton that was previously cooled to 10°C. The resulting solid was isolated by filtration under reduced pressure, washed twice with 500 mL aceton. Each filter cake was added to 5L ethanol stirred 20 hours at 50°C, isolated by filtration under reduced pressure and dried again at a temperature of 70°C under vacuum.

### Yield: 52%

The structure was confirmed by ¹³C CP MAS NMR. The product showed no peak at 45 ppm corresponding to the cellulose C6 atom of the chlorinated educt.

### ¹³C CP MAS NMR:

| δ | signal |
|---|---|
| 103 ppm | cellulose C1 |
| 90 - 65 ppm | cellulose C2-C5 |
| 60 ppm | C6_{Cellulose}-NH-***CH₂***-CH₂OH |
| 50 ppm | C6_{Cellulose}-NH-CH₂-***CH₂***NH₂ |

## Claims

1. A method for producing a modified polysaccharide, which comprises
a) providing a chlorinated polysaccharide, wherein at least a part or all of the hydroxyl groups have been replaced by chlorine atoms,
b) reacting the chlorinated polysaccharide with at least one compound, selected from compounds of the formula (I)
HX¹-R¹ (I)
wherein
X¹ is O or NR^{a}, where R^{a} is hydrogen, C₁-C₆-alkyl, C₃-C₂₀-cycloalkyl or C₆-C₂₀-aryl,
R¹ is selected from C₁-C₃₀-alkyl, C₃-C₃₀-alkenyl, C₂-C₃₀-hydroxyalkyl, amino(C₁-C₃₀-alkyl), mono-(C₁-C₆-alkyl)amino(C₁-C₃₀-alkyl), di-(C₁-C₆-alkyl)amino(C₁-C₃₀-alkyl), C₃-C₂₀-cycloalkyl and C₆-C₂₀-aryl,
wherein the alkyl group of C₁-C₃₀-alkyl and C₂-C₃₀-hydroxyalkyl may be interrupted by one or more non-adjacent groups selected from -O-, -S-, -C(=O)- and -N(R^{b})-, where R^{b} is hydrogen, C₁-C₆-alkyl, C₃-C₈-cycloalkyl or C₆-C₂₀-aryl,
wherein the cycloalkyl and aryl groups in each case can be unsubstituted or substituted by 1, 2 or 3 substituents, independently selected from hydroxy, C₁-C₆-alkyl, C₁-C₆-hydroxyalkyl, amino(C₁-C₆-alkyl), mono-(C₁-C₆-alkyl)amino(C₁-C₆-alkyl) and di-(C₁-C₆-alkyl)amino(C₁-C₆-alkyl),
to replace at least a part or all of the chlorine atoms by -X¹-R¹ groups.

2. The method according to claim 1, wherein providing the chlorinated polysaccharide in step a) comprises
a1) dissolving a polysaccharide in a solvent system which comprises at least one ionic liquid, and
a2) reacting the polysaccharide with a chlorinating agent, wherein the chlorinating agent preferably comprises or consists of thionyl chloride.

3. The method according to claim 2, wherein the polysaccharide is selected from cellulose, hemicellulose or chemically modified cellulose.

4. The method according to any of the preceding claims, wherein the chlorinated polysaccharide has a degree of substitution (DS) of at least 0.5, in particular of at least 1.

5. The method according to any of the preceding claims, wherein the chlorinated polysaccharide has a degree of polymerization (DP) of 10 to 100, preferably 20 to 60, in particular 30 to 50.

6. The method according to any of the preceding claims, wherein in step a) a chlorinated cellulose is provided, wherein 60 to 100%, preferably 80 to 100%, in particular 90 to 99.9% of the hydroxyl groups in the 6-position have been replaced by chlorine atoms.

7. The method according to any of the preceding claims, wherein in step a) a chlorinated cellulose is provided, wherein 0 to 20%, more preferably 0.1 to 10% of the hydroxyl groups directly bound to the ring carbon atoms have been replaced by chlorine atoms.

8. The method according to any of the preceding claims, wherein the compound of the formula (I) is selected from
- compounds of the formula (1.1)
HX¹¹-R¹¹ (I.1)
wherein
X¹¹ is O or NR^{a}, where R^{a} is hydrogen, C₁-C₆-alkyl, C₃-C₂₀-cycloalkyl or C₆-C₂₀-aryl,
R¹¹ is selected from C₁-C₃₀-alkyl, C₃-C₂₀-cycloalkyl and C₆-C₂₀-aryl,
wherein the cycloalkyl and aryl groups in each case can be unsubstituted or substituted by 1, 2 or 3 substituents, independently selected from hydroxy, C₁-C₆-alkyl, C₁-C₆-hydroxyalkyl, amino(C₁-C₆-alkyl), mono-(C₁-C₆-alkyl)amino(C₁-C₆-alkyl) and di-(C₁-C₆-alkyl)amino(C₁-C₆-alkyl),
- compounds of the formula (I.2)
HX¹²-R¹² (I.2)
wherein
X¹² is O or NR^{a}, where R^{a} is hydrogen, C₁-C₆-alkyl, C₃-C₂₀-cycloalkyl or C₆-C₂₀-aryl,
R¹² is selected C₂-C₃₀-hydroxyalkyl, amino(C₁-C₃₀-alkyl), mono-(C₁-C₆-alkyl)amino(C₁-C₃₀-alkyl) and di-(C₁-C₆-alkyl)amino(C₁-C₃₀-alkyl),
wherein the cycloalkyl and aryl groups in each case can be unsubstituted or substituted by 1, 2 or 3 substituents, independently selected from hydroxy, C₁-C₆-alkyl, C₁-C₆-hydroxyalkyl, amino(C₁-C₆-alkyl), mono-(C₁-C₆-alkyl)amino(C₁-C₆-alkyl) and di-(C₁-C₆-alkyl)amino(C₁-C₆-alkyl),
- mixtures thereof.

9. The method according to any of the preceding claims, wherein in step b) the compounds of the formula (I) are used in an at least 3-fold molar excess, preferably in an at least 6-fold molar excess, more preferably in an at least 10-fold molar excess over the chlorinated polysaccharide.

10. The process according to any of the preceding claims, wherein the reaction in step b) is performed in the essential absence of an extraneous solvent or wherein the reaction in step b) is performed in the presence of a solvent, preferably selected from polar aprotic solvents, ionic liquids and mixtures thereof.

11. The process according to claim 10, wherein the reaction in step b) is performed in the presence of a solvent selected from alcohols, ketones, ethers, esters, acetonitrile, ethylencarbonate, formamide, dimethylformamide, dimethylacetamide, dimethylsulfoxid and mixtures thereof.

12. The process according to any of the preceding claims, wherein the reaction in step b) is performed in the presence of a base.

13. The process according to claim 12, wherein the base is employed in at least stoichiometric amount with regard to the molar amount of the chlorine groups of the chlorinated polysaccharide.

14. A modified polysaccharide, obtainable by the process according to any of the claims 1 to 13.

15. The modified polysaccharide according to claim 14, having a degree of polymerization (DP) of 10 to 100, preferably 20 to 60, in particular 30 to 50.

16. The modified polysaccharide according to claim 14 or 15, being essentially free of chlorine atoms.

17. The use of a modified polysaccharide as defined in any one of claims 14 to 22 or obtainable by the process according to any of the claims 1 to 13
- as an intermediates that is susceptible for a further substitution, e.g. with polymerizable groups,
- as an auxiliary in pharmacy or cosmetics, preferably as or in coatings for solid drug forms,
- as surface-active compound,
- as or in (an) adhesive(s), or
- as or in (a) coating(s) for the textile, paper, printing and leather industry.
